(19) Europäisches Patentamt
European Patent Office
Office européen des brevets

(11) **EP 4 635 557 A1**

(12) **EUROPEAN PATENT APPLICATION**

(43) Date of publication:
**22.10.2025  Bulletin 2025/43**

(21) Application number: **24170565.6**

(22) Date of filing: **16.04.2024**

(51) International Patent Classification (IPC):
**A61N 1/36** (2006.01)    **A61N 1/05** (2006.01)

(52) Cooperative Patent Classification (CPC):
**A61N 1/36146;** A61B 5/346; A61N 1/0556;
A61N 1/36053; A61N 1/3611; A61N 1/36114;
A61N 1/36135; A61N 1/36157; A61N 1/36171;
A61N 1/36175; A61N 1/36178; A61N 1/36182

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Merck Patent GmbH**
**64293 Darmstadt (DE)**

(72) Inventors:
• **SCHNEIDER, Armin**
**64293 Darmstadt (DE)**
• **SCHUMACHER, Christian**
**64293 Darmstadt (DE)**

(74) Representative: **Merck Patent Association**
**Merck Patent GmbH**
**64271 Darmstadt (DE)**

(54) **SYSTEM FOR INHIBITING ACTION POTENTIAL CONDUCTION IN A NERVE**

(57)    The invention provides a system (100) for inhibiting conduction of an action potential (25) in a nerve (10), wherein the system (100) comprises an arrangement (200), wherein: the arrangement (200) comprises a tubular hosting element (211) configured for at least partially surrounding the nerve (10), wherein the tubular hosting element (211) comprises a plurality of electrodes (220); the system (100) is configured to apply in a treatment mode of the system (100) an electrical signal (20) via at least part of the plurality of electrodes (220), wherein the following applies: the electrical signal (20) has a frequency ($F_E$) selected from the range of 5 - 50 kHz; the electrical signal (20) has an amplitude ($A_E$) selected from the range of 2-10 mA; the electrical signal (20) comprises a plurality of phases (30), wherein the plurality of phases (30) comprises alternating cathodic phases (31) and anodic phases (32), wherein each phase (30) has a duration $T_P$ individually selected from the range of 5 - 60 $\mu$s; and the electrical signal (20) comprises a therapeutic period, wherein $F_E$, $A_E$ and $T_P$ are constant during the therapeutic period, and wherein during the therapeutic period the electrical signal (20) has a charge per time (CPT) selected from the range of 1.0 - 7.0 mC/s, wherein $CPT = F_E * A_E * T_P$.

EP 4 635 557 A1

**Description**

FIELD OF THE INVENTION

The invention relates to a system for inhibiting action potential conduction in a nerve. The invention further relates to a method for inhibiting action potential conduction in a nerve.

BACKGROUND OF THE INVENTION

Approaches for inhibiting action potential conduction in a nerve are known in the art. For instance, US 10441787B2 describes devices and methods in which a signal is delivered to the vagus nerve or the pulmonary branches of the vagus nerves for treating bronchoconstriction and preventing and/or ameliorating bronchoconstriction.

SUMMARY OF THE INVENTION

Electrical stimulators can be used to elicit or inhibit (or "block") the propagation of action potentials along axons in a nerve. In particular, devices that elicit action potentials may be in clinical use for a number of peripheral and cranial nerves for the treatment of various conditions (e.g., Vagus nerve stimulator for the treatment of epilepsy, depression, or to foster stroke rehabilitation, Hypoglossal nerve stimulators for the treatment of sleep apnea, Occipital nerve stimulators for the treatment of headaches). However, there may be a lack of suitable methods and devices for inhibiting action potential conduction. In particular, there may be a need for methods and devices with a high blocking efficacy combined with good safety and tolerability.

Blocking nerve signaling may be desirable for a number of diseases (or "indications"), for instance for respiratory obstructive diseases such as COPD. Patients suffering from such diseases may benefit from blocking parasympathetic pulmonary B-fibers which drive bronchoconstriction and mucus secretion.

The prior art may describe the application of direct current (DC), high-frequency alternating current Block (HF Block), and low frequency block for the blocking of action potential conduction in nerves.

DC nerve block may work by using an electrode to apply a pulsed direct current to the nerve, which may create a localized electric field that establishes a reversible conduction block. The direct current causes a hyperpolarization of the nerve membrane, deactivating voltage-gated ion channels and resulting in blockage of action potential propagation. However, the application of direct current comes with the risk of damaging tissue of the nerve or its surrounding due to changes in the tissue pH, temperature or the electrode material by electrolysis.

In contrast to DC nerve block, HF Block uses alternating current pulses delivered to the nerve at high frequencies. The HF block may initially activate sodium channels that then go into a refractory state, which may lead to a reduction in membrane excitability, blocking the propagation of action potentials throughout the application of HF Block. However, the effectiveness of HF block may be limited due to several remaining challenges: (a) difficulty in blocking of axon classes with relatively high excitation thresholds, (b) the presence of onset and offset excitation phenomena (see below), (c) discomfort to the patient due to high electrical field strength and high frequency, (d) material or tissue damage in chronic applications due to high electrical field strength and high frequency, and (e) inability to selectively block the activities of desired nerve fibers.

Low frequency block may have substantial lasting effects after the blocking signal is provided, i.e., low frequency block may have poor reversibility. In addition, low frequency blocks may have a relatively large and long-lasting (or "sustained") onset effect.

The terms "onset effect" and "offset effect" may herein refer to transiently initiating action potentials before the nerve block is established or after the block is relieved, respectively. This effect can be problematic as it can cause discomfort, pain, cardiac arrhythmias and may limit the effectiveness of the nerve block due to incomplete or variable block onset and/or offset.

Further, the prior art may describe methods and systems leading to accumulating charge disbalance (also called "DC bias"), which may cause damage to electrode materials due to irreversible electrochemical reactions of the electrode material, which may in turn lead to a loss of the needed therapeutic effect and the need to explant the device which exposes the patient to medical risks. Nerve tissue and other surrounding tissues may be permanently damaged due to strong shifts in pH or other ionic imbalances. Moreover, the accumulation of charge may lead to induction of cardiac arrhythmias and cardiac arrest even when the electrode is placed at great distance from the heart. Potentially, seizures may be induced. Furthermore, this DC bias may lead to malfunctioning of other implanted devices, e.g., cardiac pacemakers and defibrillators.

The prior art may further describe pharmacological treatments. However, depending on the indication, pharmaceutical treatment may be unavailable or provide only limited relief. For instance, for chronic obstructive pulmonary disease (COPD), pharmacological treatments may be limited, may achieve insufficient improvement in symptoms, and may not halt the progression of the disease. Moreover, available drugs for COPD such as long-acting muscarinic antagonists (LAMAs) have adverse (side) effects (e.g., mouth dryness, constipation, diarrhea, cough, headache, increase in intraocular pressure, urinary retention and others) that may lead to a low adherence to treatment or to the exclusion of patient groups also suffering from other afflictions, such as patients (also) suffering from glaucoma or prostate hyperplasia. Such adverse effects may also be dose limiting and may have led to the marketing of drugs for COPD at the lower end of clinical benefits. For drugs applied via inhalers, the inhalation of these drugs needs to be correctly performed which is often not achieved in practice and may lead to low estimates for patients' compliance in COPD. Further, also for treatments applied via pills (or other consumables), the treatment may require regular intake of the pill by the patient, and may thus rely on the attention/compliance of the patient.

The prior art may further describe nerve ablation techniques, for instance targeted lung denervation (TLD) for the treatment of COPD or pulmonary vein isolation (PVI), also called antral pulmonary vein isolation (APVI), for treating atrial fibrillation (AF). However, nerve ablation carries substantial risk of complications, and is permanent in nature.

There thus remains a need for an effective (electrical) block without major adverse effects.

Hence, it is an aspect of the invention to provide an alternative system and an alternative method for blocking action potential conduction in a nerve, which preferably further at least partly obviate one or more of above-described drawbacks. The present invention may have as object to overcome or ameliorate at least one of the disadvantages of the prior art, or to provide a useful alternative.

In a first aspect, the invention may provide a system for inhibiting action potential conduction in a nerve, especially in an axon of the nerve. The system may comprise an arrangement, such as a cuff arrangement, wherein the arrangement comprises a tubular hosting element configured for at least partially surrounding the nerve. The tubular hosting element may especially comprise a plurality of electrodes. In embodiments, the system may have a treatment mode. The system may in the treatment mode especially be configured to apply an electrical signal (to the nerve) via at least part of the plurality of electrodes. The electrical signal may have a frequency $F_E$ selected from the range of 5 - 100 kHz, such as from the range of 5 - 50 kHz, especially from the range of 10 - 35 kHz. The electrical signal may further especially be an alternating current signal. In embodiments, the electrical signal may have an (absolute) amplitude $A_E$ selected from the range of 1 - 20 mA, such as from the range of 2 - 10 mA. In further embodiments, the electrical signal may comprise a plurality of phases, especially wherein the plurality of phases comprises cathodic phases and anodic phases. The cathodic phases and the anodic phases may temporally especially be arranged alternatingly. In embodiments, each phase (of the plurality of phases) may have a duration (or "pulse width") $T_P$ individually selected from the range of 1 - 200 $\mu$s, such as from the range of 5 - 60 $\mu$s. Further, the electrical signal may comprise a therapeutic period, wherein $F_E$, $A_E$ and $T_P$ are (essentially) constant during the therapeutic period. In embodiments, during the therapeutic period, the electrical signal may have a charge per time (CPT) selected from the range of 0.25 - 7.0 mC/s, especially from the range of 1.0 - 7.0 mC/s, or especially from the range of 0.7 - 4.0 mC/s, wherein CPT = $F_E$*$A_E$*$T_P$.

In specific embodiments, the invention may provide a system for inhibiting action potential conduction in a nerve, wherein the system comprises an arrangement, wherein: the arrangement comprises a tubular hosting element configured for at least partially surrounding the nerve, wherein the tubular hosting element comprises a plurality of electrodes; the system is configured to apply in a treatment mode of the system an electrical signal via at least part of the plurality of electrodes, wherein the following applies: the electrical signal has a frequency ($F_E$) selected from the range of 5 - 100 kHz; the electrical signal has an amplitude ($A_E$) selected from the range 1 - 20 mA; the electrical signal comprises a plurality of phases, wherein the plurality of phases comprises alternating cathodic phases and anodic phases, wherein each phase has a duration $T_P$ individually selected from the range of 1 - 200 $\mu$s; and the electrical signal comprises a therapeutic period, wherein $F_E$, $A_E$ and $T_P$ are constant during the therapeutic period, and wherein during the therapeutic period the electrical signal has a charge per time (CPT) selected from the range of 0.25 - 7 mC/s, wherein CPT = $F_E$*$A_E$*$T_P$. In further embodiments, the invention may provide a system for inhibiting action potential conduction in a nerve, wherein the system comprises an arrangement, wherein: the arrangement comprises a tubular hosting element configured for at least partially surrounding the nerve, wherein the tubular hosting element comprises a plurality of electrodes; the system is configured to apply in a treatment mode of the system an electrical signal via at least part of the plurality of electrodes, wherein the following applies: the electrical signal has a frequency ($F_E$) selected from the range of 5 - 50 kHz; the electrical signal has an amplitude ($A_E$) selected from the range 2-10 mA; the electrical signal comprises a plurality of phases, wherein the plurality of phases comprises alternating cathodic phases and anodic phases, wherein each phase has a duration $T_P$ individually selected from the range of 5 - 60 $\mu$s; and the electrical signal comprises a therapeutic period, wherein $F_E$, $A_E$ and $T_P$ are constant during the therapeutic period, and wherein during the therapeutic period the electrical signal has a charge per time (CPT) selected from the range of 1.0 - 7.0 mC/s, wherein CPT = $F_E$*$A_E$*$T_P$.

The system of the invention may be suitable for effectively blocking nerve conduction in the (axon of the) fiber, while causing limited to no adverse effects. In particular, the system of the invention may result in relatively small or absent onset effects and offset effects. In particular, the stimulation parameter may enable a high frequency (or: kilohertz alternating current - KHAC) block capable of blocking action potential conduction in fibers in large mammals and humans in an effective and safe way, particularly with regards to blocking propagation of action potentials in B-fibers. The system may be used to treat patients with chronic obstructive pulmonary disease (COPD) and other obstructive pulmonary diseases, cardiac diseases and intestinal diseases, especially those characterized by increased vagal activity.

In particular, the system and method of the invention may facilitate temporarily or intermittently inactivating nerve activity, optionally based on a sensor signal, such as based on an ECG for cardiac diseases (see below).

Further, the system of the invention may facilitate selectively blocking the action potential conduction in specific nerves (see further below), thereby (essentially) not disrupting other biological processes.

Yet further, the (low) amplitude and energy requirements of the system may allow integration in an implantable device.

The invention may thus provide a system for inhibiting action potential conduction in a nerve, especially in an axon (or: "nerve fiber") thereof.

The term "nerve" may herein refer to a bundle of axons providing communication pathways from the central nervous system to peripheral organs or *vice versa.* In particular, the communication between the central nervous system and peripheral organs may occur through the conduction of action potential(s) along axons, which may be elongated components of neurons. In particular, the conduction of action potential along axons may lead to the activation of (downstream) biological processes, such as to muscle contraction. Various diseases (see further below) are characterized (in part) by hyperactivity of one or more nerves, resulting in detrimental activation of downstream biological processes. Patients suffering from such diseases may substantially benefit from the blocking of action potential conduction.

In embodiments, the system may especially comprise an arrangement, especially wherein the arrangement is configured for implantation in a subject, such as in a human subject. Hence, the arrangement may especially comprise an implantable arrangement. In particular, (external) components of the arrangement may be biocompatible.

For instance, the system may comprise a device, wherein the device comprises the arrangement, and wherein the device is configured to be at least partially implanted in the subject. Hence, (external) components of the device may be biocompatible. The device may, in embodiments, further comprise the control system, especially wherein the control system comprises or is functionally coupled to a pulse generator.

In further embodiments, the device may be configured to be implanted at the neck of the subject. Such embodiments may be particularly convenient for stimulation of a cranial nerve, especially for stimulation of the vagus nerve (also see below).

The arrangement may especially comprise a hosting element configured for at least partially surrounding a (target) nerve. As a nerve may typically have a tubular shape, the hosting element may be configured to define a tubular space for hosting the nerve. In particular, in embodiments, the hosting element may comprise a tubular hosting element, wherein the tubular hosting element is configured to define a tubular space for hosting the nerve. Especially, during use, the (tubular) hosting element may (partially) surround the nerve and be in physical contact with the nerve.

In embodiments, the tubular hosting element may have an elongated tubular shape with two openings at opposite ends. The nerve may essentially enter the tubular hosting element through one of the openings and exit the tubular hosting element through the other opening. Such embodiments may herein especially be referred to as a 'cuff arrangement'. In embodiments, the arrangement may thus comprise a cuff arrangement. In further embodiments, the cuff arrangement may comprise (multichannel) cuff electrodes, i.e., the electrodes may comprise cuff electrodes. The cuff arrangement may be beneficial in view of current insulation (i.e., to prevent current leakage), a (relatively) low strain on the nerve tissue, and ease of implantability.

In further embodiments, the tubular hosting element may have a spiral shape, especially a helical shape. The spiral shape, especially the helical shape, may define a tubular space on the 'inside' of the spirals. Hence, in such embodiments, the spirals may essentially be configured to wrap around the nerve.

The (tubular) hosting element may especially comprise an electrode, such as a plurality of electrodes. The electrode(s) may be configured to provide an electrical signal to the nerve (hosted by the hosting element). Hence, the electrode may be configured to be in electrical contact with the nerve when the hosting element is arranged (at least partially) surrounding the nerve.

The system may comprise a control system, wherein the control system is configured to control the electrode, especially the plurality of electrodes.

The term "controlling" and similar terms herein may especially refer at least to determining the behavior or supervising the running of an element. Hence, herein "controlling" and similar terms may e.g. refer to imposing behavior to the element (determining the behavior or supervising the running of an element), etc., such as e.g. measuring, displaying, actuating, opening, shifting, changing temperature, etc.. Beyond that, the term "controlling" and similar terms may additionally include monitoring. Hence, the term "controlling" and similar terms may include imposing behavior on an element and also imposing behavior on an element and monitoring the element. The controlling of the element can be done with a control system. The control system and the element may thus at least temporarily, or permanently, functionally be coupled. The element may comprise the control system. In embodiments, the control system and the element may not be physically coupled. Control can be done via wired and/or wireless control. The term "control system" may also refer to a plurality of different control systems, which especially are functionally coupled, and of which e.g. one master control system may be a control system and one or more others may be slave control systems.

The system, especially the control system, may have an operational mode, herein especially a treatment mode. The term "operational mode" may also be indicated as "controlling mode". The system, or apparatus, or device (see further also below) may execute an action in a "mode" or "operational mode" or "mode of operation". Likewise, in a method an action, stage, or step may be executed in a "mode" or "operation mode" or "mode of operation". This does not exclude that the system, or apparatus, or device may also be adapted for providing another operational mode, or a plurality of other operational modes. Likewise, this does not exclude that before executing the mode and/or after executing the mode one or more other modes may be executed. However, in embodiments, a control system (see further also below) may be available, that is adapted to provide at least the operational mode. Would other modes be available, the choice of such modes may especially be executed via a user interface, though other options, like executing a mode in dependence of a sensor signal or a (time) scheme, may also be possible. The operational mode may in embodiments also refer to a system, or apparatus, or device, that can only operate in a single operational mode (i.e. "on", without further tunability).

Hence, the system, especially a control system of the system, may have a treatment mode. In the treatment mode, the system may be configured to apply an electrical signal (to the nerve), especially via the electrode, or especially via (at least part of) the plurality of electrodes. The system, especially the control system, may thus be configured to apply the electrical signal (to the nerve) using one or more electrodes. In embodiments, the system may be configured for applying monopolar stimulation during the treatment mode, i.e., stimulation using a single electrode, such as using a single ring electrode (see below). In further embodiments, the system may be configured for applying bipolar stimulation during the treatment mode, i.e., stimulation using two electrodes, such as with two longitudinally aligned electrodes with (essentially) equal contact surface areas. In further embodiments, the system may be configured for applying tripolar stimulation during the treatment mode, i.e., stimulation using three electrodes, such as with three longitudinally aligned electrodes, wherein a central (second) electrode has (essentially) the same (contact) surface area as the other two electrodes combined (see below).

The term "stimulation", such as in "monopolar stimulation" may herein refer to the providing of an electrical signal to a nerve. The term "stimulation" herein thus does not imply an induction of nerve activity but may, in the context of the present invention, rather refer to applying an electrical signal to the nerve for the blocking of nerve activity, i.e., for the blocking of the conduction of an action potential in the nerve.

The electrical signal may have (stimulation) parameters selected for the blocking of the nerve. In particular, in embodiments, the treatment mode may comprise preventing an action potential from travelling along a neuron in the (vagus) nerve, especially by preventing the action potential to propagate beyond an area of the nerve covered by an electrode.

In embodiments, the frequency $F_E$, amplitude $A_E$ and pulse width $T_P$ may be selected to provide a therapeutic (blocking) effect during at least a therapeutic period of the electrical signal.

In further embodiments, the frequency $F_E$, amplitude $A_E$ and pulse width $T_P$ may be selected to be suitable to block an induced action potential. In particular, it will be clear to the person skilled in the art that it may be challenging to objectively determine the extent of blocking of action potential conduction in a nerve. Hence, the operational parameters may especially be selected to be suitable for blocking (part of) an induced action potential (also see below) related to relevant action potentials to be blocked during the therapeutic period. For instance, the frequency $F_E$, amplitude $A_E$ and pulse width $T_P$ may be selected suitable for blocking the conduction of induced action potentials (also see below), such as at least 10% of the induced action potentials in the totality of targeted nerve fibers, especially at least 20%, such as at least 40%, especially at least 60%. In particular, the frequency $F_E$, amplitude $A_E$ and pulse width $T_P$ may be selected such to be suitable for (completely) blocking the induced action potential conduction in at least 10% of a totality of nerve fibers during the therapeutic period, especially at least 20%, such as at least 40%, especially at least 60%. The fibers (of the totality of fibers) for which the action potential conduction is blocked may vary during the therapeutic period.

In embodiments, the electrical signal may, especially during a therapeutic period, have a frequency $F_E$, wherein the frequency $F_E$ is selected from the range of 2 - 100 kHz, such as from the range of 3 - 50 kHz, especially from the range of 5 - 25 kHz, such as from the range of 10 - 25 kHz, especially from the range of 10 - 20 kHz. In further embodiments, the frequency $F_E$ may be selected from the range of 5 - 100 kHz, such as from the range of 8 - 50 kHz, especially from the range of 10 - 35 kHz, such as from the range of 20 - 33 kHz. In further embodiments, the frequency $F_E$ may be at least 2 kHz, such as at least 3 kHz, especially at least 5 kHz, such as at least 7 kHz, especially at least 8 kHz, such as at least 10 kHz. In further embodiments, the frequency $F_E$ may be at least 15 kHz, such as at least 20 kHz. In further embodiments, the frequency $F_E$ may be at most 100 kHz, such as at most 70 kHz, especially at most 50 kHz, such as at most 35 kHz, especially at most 33 kHz. Frequencies in the range of 3 - 35 kHz, such as in the range of 10-33 kHz, especially in the range of 20 - 33 kHz may be particularly suitable for blocking action potential conduction in B fibers in the vagus nerve.

In further embodiments, the electrical signal may, especially during a therapeutic period, have an (absolute) amplitude $A_E$, wherein the amplitude $A_E$ is selected from the range of 1 - 20 mA, especially in the range of 2 - 15 mA, such as from the range of 2 - 10 mA, especially from the range of 3 - 5 mA, or especially from the range of 5 - 8 mA. In further embodiments, the amplitude $A_E$ may be selected from the range of 2.5 - 12 mA, especially form the range of 2 - 6 mA, such as

as from the range of 4 - 6 mA. In further embodiments, the amplitude $A_E$ may be at least 1 mA, such as at least 2 mA, especially at least 2.5 mA, such as at least 3 mA, especially at least 5 mA. In further embodiments, the amplitude $A_E$ may be at most 20 mA, such as at most 15 mA, like at most 12 mA, especially at most 10 mA, such as at most 8 mA, especially at most 5 mA. Amplitudes from the range of 2.5 - 12 mA, especially from the range of 5 - 8 mA may be particularly suitable.

The term "amplitude" may herein especially refer to the maximum absolute value of the difference between a peak amplitude and a reference amplitude.

In further embodiments, the electrical signal may, especially during the therapeutic period, comprise a plurality of phases. The plurality of phases may comprise cathodic phases and anodic phases. In embodiments, the cathodic phases and the anodic phases may be alternatingly arranged in time, i.e.: Cathodic-Anodic-C-A-C-A-C-A-.... Hence, the plurality of phases may comprise alternating cathodic phases and anodic phases. In further embodiments, the cathodic phases may be arranged in time such that two successive cathodic phases are followed by two successive anodic phases and *vice versa,* i.e.,: Cathodic-Cathodic-Anodic-Anodic-C-C-A-A-C-C-A-A-....

The terms "cathodic phase" and "anodic phase" herein refer to phases wherein the electrical signal has opposite sign, i.e., the sign of the electrical signal in the cathodic phase is opposite of the sign of the electrical signal in the anodic phase, wherein the sign of the cathodic phase may be negative.

In embodiments, to prevent charge accumulation, a series of successive cathodic and anodic phases may, together, be (essentially) charge balanced. In particular, in embodiments, each cathodic phase may be charged balanced with a preceding and/or subsequent anodic phase. A series of phases may herein be considered "charge balanced" when there is effectively no (net) accumulation of charge as a result of the series of phases.

Hence, in embodiments, successive cathodic phases and anodic phases may be charge-balanced. In particular, each cathodic phase may comprise providing a pulse, and each anodic phase may comprise providing a counter pulse (or *vice versa*), wherein the charge of the pulse and the counter pulse differ by less than 5%, such as by less than 3%, especially by less than 1 %, including 0%, and especially wherein the duration of the pulse and the counter pulse differ by less than 1%. Hence, in embodiments, successive cathodic phases and anodic phases may have (essentially) the same duration. In particular, in further embodiments, successive cathodic phases and anodic phases may have (essentially) the same area under curve (AUC) of charge. For instance, a control system may be configured to measure the resulting polarization of a (first) pulse and adjust the amplitude of a corresponding (second) counter pulse to control the charge balance.

It will be clear to the person skilled in the art that (net) charge accumulation may depend on several factors, such as the technical implementation of an implantable pulse generator, a neural interface, signal amplitude, signal reference level, pulse duration, et cetera. In embodiments, the operational parameters may be chosen such that an overall potential (or "DC drift") remains below 150 mV, such as below 100 mV, especially below 50 mV.

In embodiments, the system may be configured for DC offset mitigation (to address charge accumulation) via one or more of ground shunting, shunting a stimulation electrode to a counter electrode, modulation of an (anodic or cathodic) amplitude, modulation of an (anodic or cathodic) pulse width, and insertion of an extra counter pulse.

In embodiments each phase (of the plurality of phases) may, especially during the therapeutic period, have a pulse width $T_P$ (or "duration $T_P$") individually selected from the range of 3 - 120 μs, especially from the range of 4 - 90 μs, such as from the range of 5 - 60 μs, especially from the range of 10 - 50 μs, such as from the range of 15 - 50 μs. In further embodiments, each phase (of the plurality of phases) may, especially during the therapeutic period, have a pulse width $T_P$ (or "duration $T_P$") individually selected from the range of 1 - 200 μs, such as from the range of 2 - 100 μs, especially from the range of 3 - 80 μs. In further embodiments, each phase (of the plurality of phases) may have a pulse width $T_P$ of at least 1 μs, such as at least 2 μs, especially at least 3 μs, such as at least 4 μs, especially at least 5 μs, such as at least 10 μs, especially at least 15 μs. In further embodiments, each phase (of the plurality of phases) may have a pulse width $T_P$ of at most 120 μs, such as at most 90 μs, especially at most 60 μs, such as at most 50 μs, especially at most 30 μs.

In specific embodiments, the electrical signal may have a frequency $F_E$ selected from the range of 10 - 35 kHz, an amplitude $A_E$ selected from the range of 5 - 8 mA, and for each phase (of the plurality of phases) the duration $T_P$ may be individually selected from the range of 15 - 50 μs. In further embodiments the electrical signal may have a frequency $F_E$ selected from the range of 10 - 35 kHz, an amplitude $A_E$ selected from the range of 2.5 - 12 mA, and for each phase (of the plurality of phases) the duration $T_E$ may be individually selected from the range of 3 - 80 μs.

As described above, the electrical signal may comprise a therapeutic period. The term "therapeutic period" may herein especially refer to a (time) period during which the electrical signal has parameters suitable for providing a therapeutic effect to the nerve, especially wherein the therapeutic effect is a blocking of action potential conduction in the nerve. In particular, the electrical signal may comprise gap periods and/or adjustment periods (for one or more parameters), during (part of) which essentially no therapeutic effect is present. In embodiments, the electrical signal may also comprise a plurality of therapeutic periods. Successive therapeutic periods may be temporally separated or may be consecutive (see below). In embodiments, the frequency $F_E$, amplitude $A_E$ and pulse width $T_P$ may (each) be essentially constant during the therapeutic period. In particular, each of the frequency $F_E$, amplitude $A_E$ and pulse width $T_P$ may typically vary less than 5% (relative to a maximal value) during the therapeutic period, such as less than 3%, especially less than 1%, such as (essentially) 0%. The pulse width $T_P$ being (essentially) constant during the therapeutic period herein refers to all (cathodic and anodic) phases occurring during the therapeutic period having (essentially) the same pulse width $T_P$. Hence, the therapeutic period may be a period during which (a) there is a therapeutic effect (see above) and (b) the operational parameters are (essentially) constant).

As described above, a control system configured for charge balancing may, if needed, (occasionally) modify the amplitude and/or the pulse width of a pulse to prevent charge accumulation. Hence, in further embodiments, each of the frequency $F_E$, amplitude $A_E$ and pulse width $T_P$ may vary less than 20% (relative to a maximal value) during the therapeutic period, such as less than 10%, especially less than 5%. In further embodiments, during at least 80%, especially at least 90%, of a therapeutic period, each of the frequency $F_E$, amplitude $A_E$ and pulse width $T_P$ may vary less than 5% (relative to a maximal value) during the therapeutic period, such as less than 3%, especially less than 1%, such as (essentially) 0%.

In further embodiments, during the therapeutic period, the electrical signal may have a charge per time CPT selected from the range of 0.25 - 7.0 mC/s, such as from the range of 1.0 - 7.0 mC/s, or such as from the range of 0.75 - 6.5 mC/s, especially from the range of 1.0 - 6.0 mC/s. In further embodiments, during the therapeutic period, the electrical signal may have a charge per time CPT selected from the range of 0.4 - 5.0 mC/s, especially from the range of 0.5 - 4.0 mC/s, such as from the range of 0.7 - 3.0 mC/s, such as from the range of 0.8 - 2.0 mC/s, especially from the range of 0.9 - 1.5 mC/s, wherein CPT = $F_E*A_E*T_P$. The inventors have surprisingly found that an electrical signal having a charge per time in such range provides an effective blocking effect while having limited to no onset and offset effects (see below).

In embodiments wherein the electrical signal comprises a plurality of therapeutic periods, especially for each therapeutic period may apply that CPT is (individually) selected from the range of 0.25 - 7.0 mC/s, such as from the range of 1.0 - 7.0 mC/s, or such as from the range of 0.75 - 6.5 mC/s, especially from the range of 1.0 - 6.0 mC/s. In further embodiments, for each therapeutic period may apply that CPT is (individually) selected from the range of 0.5 - 4.0 mC/s, such as from the range of 0.7 - 4.0 mC/s, such as from the range of 0.8 - 2.0 mC/s, especially from the range of 0.9 - 1.5 mC/s.

In further embodiments, during the therapeutic period, especially during each therapeutic period, CPT may be at least 0.25 mC/s, especially at least 0.5 mC/s, such as at least 0.6 mC/s, especially at least 0.7 mC/s, such as at least 0.75 mC/s. In further embodiments, during the therapeutic period, especially during each therapeutic period, CPT may be at least 0.8 mC/s, especially at least 0.9 mC/s, such as at least 1.0 mC/s, especially at least 1.2 mC/s, such as at least 1.5 mC/s. In further embodiments, during the therapeutic period, especially during each therapeutic period, CPT may be at most 7.0 mC/s, especially at most 6.5 mC/s, such as at most 6.0 mC/s. In further embodiments, during the therapeutic period, especially during each therapeutic period, CPT may be at most 5.0 mC/s, especially at most 4.0 mC/s, such as at most 3.0 mC/s, especially at most 2.5 mC/s, such as at most 2.0 mC/s, especially at most 1.5 mC/s.

As described above, in embodiments the therapeutic periods may be arranged consecutively, i.e., with (essentially) no time in between, such as ≤ 30 s between them, especially ≤ 20 s, such as ≤ 10 s, especially ≤ 5 s, including (essentially) 0 s. In some literature, the totality of therapeutic periods and interspersed pauses is called a duty cycle, often expressed as percent of total time. In other literature, such therapeutic periods may be referred to as bursts. For instance, in embodiments, the electrical signal may comprise n consecutively arranged therapeutic periods. In such embodiments, the successive therapeutic periods may vary in one or more of the frequency $F_E$, the amplitude $A_E$ and the pulse width $T_P$. In particular, in such embodiments, an overall (or average) CPT of the consecutively arranged therapeutic periods may be selected from the range of 0.25 - 7.0 mC/s, especially from the range of 1.0 - 7.0 mC/s, or especially from the range of 0.75 - 6.5 mC/s, such as from the range of 1 - 6.0 mC/s. In further embodiments, an overall (or average) CPT of the consecutively arranged therapeutic periods may be selected from the range of 0.5 - 4.0 mC/s, especially from the range of 1.5 - 4.0 mC/s, such as from the range of 2.0 - 3.5 mC/s, or especially from the range of 1.0 - 3.0 mC/s, such as from the range of 1.5 - 2.5 mC/s, or especially from the range of 0.7 - 3 mC/s, such as from the range of 0.8 - 2.0 mC/s, like especially from the range of 0.9 - 1.5 mC/s. Further, in such embodiments, the overall CPT of the consecutively arranged therapeutic periods may be at least 0.25 mC/s, especially at least 0.5 mC/s, such as at least 0.6 mC/s, especially at least 0.7 mC/s, such as at least 0.75 mC/s, especially at least 0.8 mC/s. In further embodiments, the overall CPT of the consecutively arranged therapeutic periods may be at least 1.0 mC/s, such as at least 1.2 mC/s, especially at least 1.5 mC/s. In further embodiments, the overall CPT of the consecutively arranged therapeutic periods may be at most 7.0 mC/s, such as at most 6.5 mC/s, especially at most 6.0 mC/s. In further embodiments, the overall CPT of the consecutively arranged therapeutic periods may be at most 5.0 mC/s, especially at most 4.0 mC/s, such as at most 3.0 mC/s, especially at most 2.5 mC/s, such as at most 2.0 mC/s, especially at most 1.5 mC/s. In particular, for n consecutive therapeutic periods the overall (also may be called average) CPT of the consecutive periods may be determined according to the following formula:

$$ CPT = \frac{\sum_{i=1}^{n} F_{E,i} * |A_{E,i}| * T_{P,i} * T_{T,i}}{\sum_{i=1}^{n} T_{T,i}} $$

wherein $F_{E,i}$, $A_{E,I}$, $T_{P,i}$, and $T_{T,I}$ are the frequency, amplitude, pulse width and therapeutic period duration of the $i^{th}$

therapeutic period, respectively.

In further embodiments, the electrical signal may have a charge density $D_E$ selected from the range of 0.002 $mC/cm^2$ - 1.2 $mC/cm^2$, such as from the range of 0.005 $mC/cm^2$ - 0.6 $mC/cm^2$, especially from the range of 0.01 $mC/cm^2$ - 0.3 $mC/cm^2$, such as from the range of 0.02 $mC/cm^2$ - 0.2 $mC/cm^2$

As described above, the system may be applicable for the preventing and/or treating of disease in a subject, such as in a (human) patient.

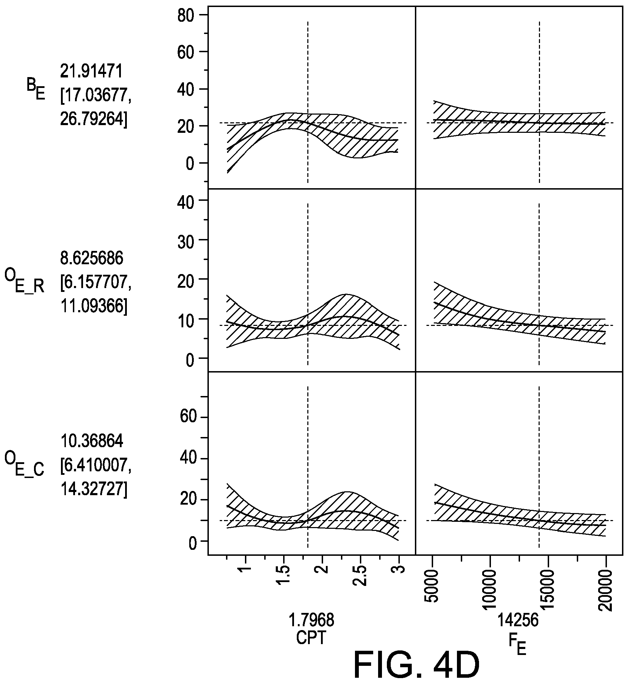

Blocking nerve signaling may be a desirable therapy for obstructive airway diseases. For instance, it may be desired to block parasympathetic pulmonary B-fibers in the vagus nerve which drive bronchoconstriction and mucus secretion, which may be key pathophysiological features of these diseases. As described below in the context of experiment 2 (also see Fig. 4D), the system and method of the invention may facilitate blocking the conduction of action potentials related to airway resistance.

Hence, in embodiments, the disease may comprise a respiratory disease (or "obstructive airway disease") selected from the group comprising chronic obstructive pulmonary disease (COPD), asthma, sleep apnea, asthma-COPD overlap syndrome (ACOS, also referred to as "ACO"), cystic fibrosis, interstitial lung disease, lung fibrosis, and pulmonary hypertension, especially primary pulmonary hypertension, or especially secondary pulmonary hypertension. In such embodiments, the (to be inhibited) action potential conduction may be related to one or more of bronchoconstriction, mucus hypersecretion and inflammation.

As described in the 2024 report of the Global Initiative for Chronic Obstructive Lung Disease (GOLD): "COPD is a heterogeneous lung condition characterized by chronic respiratory symptoms (dyspnea, cough, sputum production, exacerbations) due to abnormalities of the airways (bronchitis, bronchiolitis) and/or alveoli (emphysema) that cause persistent, often progressive airflow obstruction. In embodiments, the disease may comprise COPD.

As described in the 2023 report of the Global Initiative for Asthma: "Asthma is a heterogeneous disease, usually characterized by chronic airway inflammation. It is defined by the history of respiratory symptoms, such as wheeze, shortness of breath, chest tightness and cough, that vary over time and in intensity, together with variable expiratory airflow limitation." Additional symptoms may include exercise limitations due to bronchoconstriction and excessive mucus production. Asthma may commonly be triggered by allergens and is associated with airway hyper-responsiveness. In embodiments, the disease may comprise asthma.

Sleep apnea (also called the "overlap syndrome") may partially overlap with COPD in symptomatology. In embodiments, the disease may comprise sleep apnea.

Asthma-COPD overlap syndrome has been recognized as a separate disease entity, i.e., separate from asthma and COPD, but shows similarities in symptomatology. In embodiments, the disease may comprise Asthma-COPD overlap syndrome.

Cystic fibrosis, interstitial lung disease, and lung fibrosis are all lung diseases involving an obstructive component. In embodiments, the disease may comprise one or more of cystic fibrosis, interstitial lung disease, and lung fibrosis, especially cystic fibrosis, or especially interstitial lung disease, or especially lung fibrosis.

Pulmonary hypertension (PH) may be a complex and progressive medical condition characterized by elevated mean pulmonary arterial pressure (mPAP) at rest, exceeding 20 mmHg. It may occur due to structural and/or functional changes in the pulmonary arteries, leading to increased pulmonary vascular resistance (PVR) and impaired blood flow through the pulmonary circulation. PH may be classified into primary and secondary PH based on the underlying etiology and pathophysiology. Primary PH can be hereditary, or linked to connective tissue diseases, HIV infection, congenital heart diseases, portal hypertension, drugs/toxins, or schistosomiasis. Secondary pulmonary hypertension may occur as a consequence of conditions such as chronic lung diseases (like chronic obstructive pulmonary disease or interstitial lung disease), heart diseases (such as left-sided heart failure or valvular diseases), blood clotting disorders, or chronic high-altitude exposure. Symptoms of PH can include dyspnea, fatigue, chest pain, syncope, palpitations, and signs of right heart failure. Blocking vagal parasympathetic B-fibers may facilitate lowering airway resistance and increasing compliance (see experiment 2). The relationship between airway resistance and PH can be two-fold: In primary PH, peripheral airway obstruction has been observed as a consequence of PAH, and alleviation of bronchoconstriction by blocking pulmonary parasympathetic B-fibers is expected to improve symptoms in primary PH. In secondary PH, increased airway resistance (such as in COPD) may be a direct or indirect cause of PH, and by lowering bronchoconstriction progression of PH may be halted or even reversed. In embodiments, the disease may comprise pulmonary hypertension.

In embodiments, the (to be inhibited) action potential conduction may be involved in eliciting or worsening bronchoconstriction. Bronchoconstriction may be driven by acetylcholine release by the parasympathetic B-fibers in the vagus nerve and cholinergic activation of muscarinic receptors on the smooth musculature of the lungs. Thereby, the blocking of action potential conduction in such fiber(s) can repress bronchoconstriction, which may substantially improve patient comfort and outcomes.

In further embodiments, the (to be inhibited) action potential conduction may be involved in eliciting or worsening hypersecretion. Mucus hypersecretion may be driven by acetylcholine release by the parasympathetic B-fibers in the vagus nerve and cholinergic activation of muscarinic receptors on the mucus glands and secretory cells within the airways of the lung. Mucus hypersecretion may be considered a key pathophysiological feature of COPD. Thereby, the blocking of action potential conduction in such fiber(s) can repress mucus hypersecretion, which may substantially improve patient comfort and outcomes.

In further embodiments, the (to be inhibited) action potential conduction may be involved in eliciting or worsening local inflammation in the lungs. Acetylcholine may have a pro-inflammatory role in the lungs, possibly via the M3 receptor. By limiting cholinergic inflow into the lungs, local inflammation in diseases like COPD may thus be decreased, which may in turn improve patient outcomes.

Other examples of diseases that may be treated and/or prevented by blocking of parasympathetic (cardiac) B-fibers include: vagally mediated paroxysmal atrial fibrillation (AF), which may provide a high risk of stroke, symptomatic clinical bradyarrhythmias caused by vagal overactivity such as vasovagal syncope (VVS), sinus node dysfunction (SND), and functional atrioventricular block (AVB), which often requires cardioneuroablation therapy.

Hence, in embodiments, the system may be (configured) for preventing and/or treating a disease in a subject, wherein the disease comprises a cardiac disease selected from the group comprising atrial fibrillation, especially paroxysmal atrial fibrillation, symptomatic clinical bradyarrhythmia, vasovagal syncope, sinus node dysfunction, and functional atrioventricular block. The vagus nerve may be causally involved (in a subset of the cases) in sinus node dysfunction, and bradycardia, arrythmia and vasovagal syncope may (each) be a symptom of such malfunctioning. In particular, the mentioned (cardiac) diseases may be caused or worsened by a hyperactivity or pathological activity of the vagus nerve. In such embodiments, the (to be inhibited) action potential conduction may thus especially be related to hyperactivity (or pathological activity) in the vagus nerve.

Atrial fibrillation (AF) is the most common cardiac arrhythmia and is associated with increased morbidity and mortality, predominantly caused by stroke, myocardial infarction, and heart failure. Pharmacological AF therapy has moderate efficacy and safety issues, such as proarrhythmia and bleeding complications. At least one third of paroxysmal AF may be due to abnormal vagal activity or overactivity. Various ablation techniques exist, but may carry risk of complications, and are permanent in nature. Known complications of ablation techniques include PV stenosis, cardiac tamponade, stroke, atrioesophageal fistula, phrenic nerve injury, post procedure arrhythmias, coronary artery damage, air embolism, vascular complications, radiation injury, mitral valve trauma and periesophageal vagal injury.

Vasovagal syncope, also known as neurocardiogenic syncope or reflex syncope, refers to a common type of fainting spell characterized by a temporary loss of consciousness. It may occur due to a sudden drop in heart rate and blood pressure, resulting in insufficient blood flow to the brain. This syncope episode is often triggered by specific stimuli or situations that activate the vagus nerve. Common triggers may include emotional stress, pain, prolonged standing, heat exposure, or sudden changes in body position. During a vasovagal syncope episode, the vagus nerve may be overly stimulated, which may lead to a reflex response that slows down the heart rate and widens blood vessels. As a result, blood flow to the brain decreases, causing a brief loss of consciousness. Symptoms leading up to the fainting episode may include lightheadedness, dizziness, pallor, sweating, and nausea.

Sinus node dysfunction (SND), also known as sinus node dysfunction syndrome or sick sinus syndrome, refers to a group of heart rhythm disorders characterized by abnormal functioning of the sinus node. In sinus node dysfunction, the sinus node fails to discharge electrical signals at a regular pace, resulting in an irregular heartbeat or pauses in the heart's normal rhythm. This can lead to symptoms such as dizziness, shortness of breath, fatigue, fainting, or even loss of consciousness.

The invention may herein for explanatory purposes primarily be described in the context of treating and/or preventing respiratory and cardiac diseases by blocking the conduction of action potentials in B-fibers, especially in B-fibers in the vagus nerve. It will be clear to the person skilled in the art that the invention is not limited to such embodiments and may also serve to (a) treat and/or prevent other diseases, (b) block action potentials in A and/or C fibers, and (c) block action potentials in other nerves.

As will be known to the person skilled in the art, fibers (in the human body) may be classified into different types relating to their diameter, degree of myelination, and conduction properties. Herein, the fibers are referred to according to their classification into A, B, and C fibers in the Erlanger Gasser classification, such as described in VARGA I and MRAVEC B, Chapter 8 - Nerve Fiber Types, Nerves and Nerve Injuries, Academic Press, 2015, Pages 107-113, ISBN 9780124103900, which is hereby herein incorporated by reference. As will be known to the person skilled in the art, in the Erlanger Gasser classification, A fibers are further subdivided into different types ($A\alpha$, $A\beta$, $A\gamma$, $A\delta$) based on their size and conduction velocity. B-fibers have relatively smaller diameters and slower conduction velocities compared to A fibers. C fibers, on the other hand, may be unmyelinated and may have the smallest diameter and slowest conduction velocity among the three types.

In embodiments, the treatment mode may comprise applying the electrical signal to at least one B-fiber in the nerve. Especially, in further embodiments, the B-fiber may comprise a pulmonary B-fiber. In such embodiments, for instance, the electrical signal may be configured (or "parameterized") for blocking nerve conduction in the B-fiber such that airway resistance in the subject is decreased.

In further embodiments, the nerve may comprise a cranial nerve, especially a vagus nerve.

In further embodiments, the nerve comprises a vagus nerve, and wherein the treatment mode comprises applying the electrical signal to at least one B-fiber in the nerve.

As described above, the system of the invention may be operated temporarily or intermittently. For instance, the treatment mode be activated based on a temporal pattern, e.g., based on a time of day, or based on a sensor signal, such as an aberrant ECG measurement.

Hence, in embodiments, the control system (see above) may be configured to control the (initiation of the) treatment mode based on a temporal pattern, such as based on a time of day. For instance, the latter could be relevant for diseases that primarily provide issues during daytime or, by contrast, at night.

In further embodiments, the control system may be configured to control the (initiation of the) treatment mode based on a sensor signal. For instance, in embodiments, the system may comprise a sensor configured to detect a biological parameter and to provide a related sensor signal to the control system, wherein the control system is configured to control the (initiation of the) treatment mode based on a sensor signal. The biological parameter may, for instance, be selected from the group comprising a body temperature, a heart rate, a blood pressure, a blood oxygen level, a skin impedance, a transthoracic impedance measurement, et cetera. It will be clear to the person skilled in the art that different (combinations of) parameters may be relevant for different diseases.

The term "related sensor signal" may herein refer to a signal that is related to the detected biological parameter. In particular, the related sensor signal may comprise raw and/or processed data related to the (detected) biological parameter.

In further embodiments, the control system may be configured to control the (initiation of the) treatment mode based on a user input. For instance, in embodiments, the system may comprise a user interface configured to receive a user input, wherein the control system is configured to control the (initiation of the) treatment mode based on the user input. The user may, for example, provide an input indicating that treatment is needed, such as due to discomfort felt by the user, but could also provide input related to activities, such as informing the control system of physical activity, food intake and/or going to bed. Such latter inputs may also be relevant in combination with above-mentioned sensor option as some changes in biological parameters may be expected in line with the activities, e.g., an elevated heart rate may be expected during physical activity.

The treatment mode may thus be specifically applied to counteract ongoing or expected (elevated) action potential conduction, for instance in relation to sporadic events, but may also be regularly applied according to a treatment schedule. Accordingly, the duration of the electrical signal may be selected in view of the specific needs of the subject, and may thus substantially vary for different subjects, particularly for subjects suffering from different diseases.

In embodiments, the electrical signal may have a signal duration $T_E$, wherein $T_E$ is selected from the range of 1 s - 24 h, especially from the range of 10 s - 24 h, such as from the range of 30 s - 12 h. In further embodiments, $T_E$ may be selected from the range of 5 s - 18 h, such as from the range of 10 s - 12 h. In further embodiments, $T_E$ may be selected from the range of 1 s - 1200 s, such as from the range of 5 s - 600 s, especially from the range of 10 s- 300 s,

In further embodiments, the system may be operated (essentially) continuously, such as (on average) for at least 20 hours a day, especially (on average) at least 22 hours a day, such as (on average) at least 23 hours a day, including 24 hours.

The electrical pulse may essentially have any pulse shape. In embodiments, the electrical signal, especially a pulse and a counter pulse, may have a (symmetric) rectangular pulse shape, especially a square pulse shape. In further embodiments, the electrical signal may have a sinusoidal or triangular pulse shape.

The hosting element, especially the tubular hosting element, may be elongated along a longitudinal axis A (of the hosting element). In particular, as nerves may have wire-like shapes, i.e., nerves may have a length substantially exceeding their cross-sectional diameter, the hosting element may (also) have a length substantially exceeding its cross-sectional diameter. For instance, in embodiments, the hosting element may have an (inner) diameter $D_H$ and a length $L_H$, especially wherein the diameter $D_H$ is defined perpendicular to the longitudinal axis A and wherein the length is defined along the longitudinal axis A. The diameter $D_H$ may especially be an average equivalent circular diameter of the hosting element.

The equivalent circular diameter (or ECD) (or "circular equivalent diameter") of an (irregularly shaped) two-dimensional shape is the diameter of a circle of equivalent area. For instance, the equivalent circular diameter of a square with side a is $2*a*SQRT(1/\pi)$. For a circle, the diameter is the same as the equivalent circular diameter. Would a circle in an xy-plane with a diameter Dc be distorted to any other shape (in the xy-plane), without changing the area size, then the equivalent circular diameter of that shape would be Dc.

In embodiments, the diameter $D_H$ may be selected from the range of 1 - 9mm, such as from the range of 1.5-8 mm. The diameter $D_H$ may, when the hosting element is in a resting state, especially be selected from the range of $0.7 * D_N - 1.3 * D_N$, where $D_N$ is the nerve diameter (at the site where the hosting element is to be arranged). The nerve diameter may (also) be an average circularly equivalent diameter of the nerve (at the site where the hosting element is to be arranged).

In further embodiments, the length $L_H$ may be selected from the range of 5 - 50 mm, such as from the range of 5 - 40 mm.

In embodiments, the (tubular) hosting element may have an (inner) diameter $D_H$, wherein along the longitudinal axis A (of the tubular hosting element) each electrode has a length L (or "electrode length L") individually selected from the range of $0.1*D_H - 2*D_H$, especially from the range of $0.1*D - 0.5*D_H$. In further embodiments circumferentially around the longitudinal axis A each electrode may have a width W (or "electrode width W") individually selected from the range of $0.1*D_H - 2*D_H$, especially from the range of $0.1*D_H - 0.75*D_H$ mm.

In further embodiments, the electrodes may have lengths L individually selected from the range of 0.1 - 1.5 mm, such as from the range of 0.2 - 1 mm, especially from the range of 0.3 - 0.7 mm.

In further embodiments, the electrodes may have widths W individually selected from the range of 0.4 - 2 mm, such as from the range of 0.5 - 1.5 mm, especially from the range of 0.7 - 0.9 mm.

During use, the longitudinal axis of the (tubular) hosting element may be aligned with a direction of elongation of the nerve. In particular, the nerve may be arranged in the hosting element along the longitudinal axis, i.e., the longitudinal axis A of the tubular hosting element may, during use, be aligned with a longitudinal dimension of the nerve.

In embodiments, the arrangement may comprise a plurality of electrodes. In particular, the arrangement may comprise a biocompatible carrier substrate, wherein the biocompatible carrier substrate comprises openings, wherein the electrodes are configured to contact the nerve through the openings.

Especially, the electrodes may comprise a first electrode and a second electrode, especially (a plurality of) first electrodes and (a plurality of) second electrodes. The first electrode(s) and the second electrode(s) may along the longitudinal axis A be separated by a first edge-to-edge distance di. In further embodiments, $d_1$ may be selected from the range of 0.1 - 10 mm, such as from the range of 0.2 - 5 mm, especially from the range of 0.5 - 3 mm, such as from the range of 0.8 - 2.5 mm, especially from the range of 0.8 - 1.2 mm.

In embodiments, the system, especially the arrangement, may be configured for bipolar stimulation, wherein the longitudinally aligned first electrodes and second electrodes have (essentially) equal surface area, wherein a first edge-to-edge distance $d_1$ is selected from the range of 0.8 to 1.2 mm, wherein the electrodes have widths W selected from the range of 0.7 to 0.9 mm, and wherein the electrodes have lengths L selected from the range of 0.3 to 0.7 mm. Such embodiments may be particularly suitable for providing a successful block of action potential conduction with a low onset excitation.

In further embodiments, the electrodes may further comprise a third electrode. In such embodiments, the second electrode may be arranged between the first electrode and the third electrode. Especially, the second electrode(s) and the third electrode(s) may along the longitudinal axis A be separated by a second edge-to-edge distance $d_2$. In further embodiments, $d_2$ may be selected from the range of 0.1 - 10 mm, such as from the range of 0.2 - 5 mm, especially from the range of 0.5 - 3 mm, such as from the range of 0.8 - 2.5 mm.

As the distance between the electrodes may influence the blocking effect (see below), it may be advantageous for the first distance and the second distance to differ, such that, depending on the desired result of the electrical signal, three different distances can be selected, i.e., di, $d_2$, and $d_1+d_2+L_2$, wherein $L_2$ is the length of the second electrode. Hence, in embodiments, $d_1 \neq d_2$. Especially, $|d_1-d_2| \geq 0.1$ mm, such as $\geq 0.2$ mm, especially $\geq 0.5$ mm.

For instance, in embodiments, the first electrode and the second electrode may comprise ring electrodes. In particular, in embodiments, the (first) electrode may comprise a first ring electrode configured to surround the nerve when the hosting element hosts the nerve. Similarly, in embodiments, the second electrode may comprise a second ring electrode configured to surround the nerve when the hosting element hosts the nerve.

In further embodiments, the third electrode may (also) comprise a ring electrode, i.e., the third electrode may comprise a third ring electrode configured to surround the nerve when the hosting element hosts the nerve.

As described above, the electrodes may also comprise (a plurality of) first electrodes and (a plurality of) second electrodes. In such embodiments, the first electrodes may be circumferentially arranged in the (tubular) hosting element in a first electrode array. Similarly, in further embodiments, the second electrodes may be circumferentially arranged in the (tubular) hosting element in a second electrode array. In such embodiments, the first electrode array and the second electrode array, especially the electrodes of the first electrode array and the second electrode array, may be separated by the first edge-to-edge distance $d_1$ along the longitudinal axis A. As mentioned above, in embodiments, $d_1$ may be selected from the range of 0.1 - 10 mm, such as from the range of 0.2 - 5 mm, especially from the range of 0.5 - 3 mm, such as from the range of 0.8 - 2.5 mm.

Hence, in embodiments, the first electrodes may be essentially aligned along the circumference of the (tubular) hosting element, i.e., during use the first electrodes may be aligned along the circumference of the nerve. Similarly, in embodiments, the second electrodes may be essentially aligned along the circumference of the (tubular) hosting element.

Having multiple first and second electrodes provides flexibility in selecting the electrodes that provide the electrical signal. Thereby, specific fibers in the nerve may be selectively targeted, which may provide improved specificity and efficiency, while reducing the likelihood of side effects.

In further embodiments, the electrodes may comprise (a plurality of) third electrodes. In such embodiments, the third electrodes may especially (also) be circumferentially arranged in the (tubular) hosting element in a third electrode array. In such embodiments, the second electrode array may be arranged between the first electrode array and the third electrode array. Further, in such embodiments, the second electrode array and the third electrode array may be separated by a second edge-to-edge distance $d_2$ along the longitudinal axis A. In further embodiments, $d_2$ may be selected from the range of 0.1 - 10 mm, such as from the range of 0.2 - 5 mm, especially from the range of 0.5 - 3 mm, such as from the range of 0.8 - 2.5 mm.

As described above, in embodiments, $d_1 \neq d_2$. Especially, $|d_1-d_2| \geq 0.1$ mm, such as $\geq 0.2$ mm, especially $\geq 0.5$ mm.

In further embodiments, at least part of the first electrodes may be aligned with (respective) second electrodes. In particular, in embodiments, for each first electrode (of a first subset of the first electrodes may apply that: the first electrode is longitudinally aligned with a (respective) second electrode (of a second subset of the second electrodes). In such embodiments, it may be particularly beneficial when surface areas, especially contact surface areas of, of the (aligned) electrodes are (essentially) the same, forming a symmetric pair of blocking sites along the nerve fibers. These sites may facilitate delivering an identical charge density to the nerve with rapidly alternating cathodes as points of excitation. Hence, in further embodiments, for each first electrode (of a first subset of the first electrodes may apply that: the (contact) surface areas of the first electrode and of the second electrode differ by less than 10 %, especially less than 5%, such as less than 3 %, especially less than 1 %, including (essentially) 0 %.

The difference in (contact) surface area may be determined relative to the largest (contact) surface area of the respective electrodes.

The term 'contact surface area' may herein refer to the area of the electrode that, during use, will be in (physical) contact with the nerve. For instance, in embodiments, part of a surface area of the electrode may be a contact surface area in (physical) contact with the nerve, whereas a (non-conductive) material may be arranged between the nerve and another part of the surface area of the electrode.

Similarly, in embodiments, for each second electrode (of a second subset of the second electrodes) may apply that: the second electrode is longitudinally aligned with a (respective) third electrode (of a third subset of the third electrodes), especially wherein (contact) surface areas of the second electrode and of the third electrode differ by less than 10 %, especially less than 5 %, such as less than 3 %, especially less than 1 %, including (essentially) 0 %.

In further embodiments, longitudinally aligned electrodes, such as first electrodes and second electrodes, and/or such as second electrodes and third electrodes, may have (essentially) equal lengths (L) and widths (W). In particular, in embodiments, longitudinally aligned electrodes may have (essentially) the same aspect ratio. In embodiments, the aspect ratio of longitudinally aligned electrodes differs less than 10 %, especially less than 5 %, such as less than 3%, including (essentially) 0%.

Alternatively, in embodiments wherein the electrodes comprise first, second and third electrode arrays, second electrodes maybe enlarged relative to the first and third electrodes. In particular, the electrodes may be configured such that, during use, the second electrodes may function as anode (or cathode), while the first and third electrodes together function as cathode (or anode). In such embodiments, it may be beneficial for the second electrode to have (essentially) the same (contact) surface area as the first electrode and the third electrode combined.

Hence, in embodiments, for each second electrode (of a second subset of the second electrodes) applies that: the second electrode is longitudinally aligned with a (respective) first electrode (of a first subset of the first electrodes) and with a (respective) third electrode (of a third subset of the third electrodes), wherein the (contact) surface area of the second electrode differs less than 10 % from combined (contact) surface areas of the first electrode and the third electrode, especially less than 5 %, such as less than 3 %, especially less than 1 %, including (essentially) 0 %.

In further embodiments, the first electrode array may comprises $n_1$ first electrodes. In embodiments, $n_1$ may be selected from the range of 1 - 12, such as from the range of 2 - 10, especially from the range of 3 - 9, such as from the range of 4 - 8. In further embodiments, $n_1 \geq 3$, such as $\geq 4$, especially $\geq 6$. In further embodiments, $n_1 \leq 12$, such as $\leq 10$, especially $\leq 8$. In further embodiments, $n_1$ may be selected from the range of 4 - 12.

Similarly, in embodiments, the second electrode array may comprises $n_2$ second electrodes. In embodiments, $n_2$ may be selected from the range of 1- 12, such as from the range of 2 - 10, especially from the range of 3 - 9, such as from the range of 4 - 8. In further embodiments, $n_2 \geq 3$, such as $\geq 4$, especially $\geq 6$. In further embodiments, $n_2 \leq 12$, such as $\leq 10$, especially $\leq 8$.

As described above, the first and second electrodes may, in embodiments, (all) be aligned. Hence, in embodiments, the first subset may comprise $n_1$ electrodes, the second subset may comprise $n_2$ electrodes, and $n_1 = n_2$. Especially, in specific embodiments, $n_1 = n_2 = 6$.

In further embodiments, the third electrode array may comprise $n_3$ third electrodes. In embodiments, ns may be selected from the range of 1 - 12, such as from the range of 2 - 10, especially from the range of 3 - 9, such as from the range of 4 - 8. In further embodiments, $n_3 \geq 3$, such as $\geq 4$, especially $\geq 6$. In further embodiments, $n_3 \leq 12$, such as $\leq 10$, especially $\leq 8$.

In further embodiments, the second subset may comprise $n_2$ electrodes, the third subset may comprise $n_3$ electrodes, and $n_2 = n_3$. In further embodiments, $n_1 = n_2 = n_3$.

The electrodes may comprise essentially any biocompatible electrode material. In embodiments, the electrodes may especially comprise a high charge-injection material selected from the group comprising platinum iridium oxide and reduced graphene oxide. High charge-injection materials may be relatively robust to DC bias, and may thus be relatively tolerant to accumulating charge, and may therefore also be safer for the surrounding biological tissue (as electrode damage may lead to tissue damage).

As described above, the electrical signal may comprise cathodic phases and anodic phases. In embodiments, the cathodic and anodic phases may be successively arranged (without intervening gaps). For instance, the phases may be arranged as follows: Cathodic-Anodic-Cathodic-Anodic-C-A-C-A-.... The absence of gaps (or gaps with short durations) may have the benefit that the amplitude may be kept (relatively) low (depending on pulse width), which may lower energy consumption.

In embodiments, the electrical signal may further comprise gap phases, especially wherein the gap phases are interspersed between successive cathodic phases and anodic phases. For instance, the phases may be arranged as follows: Cathodic-Gap-Anodic-G-C-G-A-G-..., or as follows: Cathodic-Gap-Anodic-G-A-G-C-G-C-G-A-.... Hence, in embodiments, the electrical signal further comprises gap phases, wherein the gap phases are interspersed between successive cathodic phases and anodic phases (including between successive anodic phases and thereupon following cathodic phases), and, in embodiments where cathodic phases (and anodic phases) may be grouped together in sets of two, in between (each set of) two successive anodic phases and in between (each set of) two successive cathodic phases. The gap phases may facilitate charge balancing and/or recovery of the nerve fibers in between successive cathodic and anodic phases.

In the example above, both cathodic phases and anodic phases are followed by gap phases. Specifically, gap phases are included between each set of two successive cathodic and anodic phases. However, other arrangements are also possible. For instance, in further embodiments, the gap phases may only follow cathodic phases or only follow anodic phases. In yet further embodiments, the gap phases may be sporadically or periodically arranged between the cathodic and the anodic phases. For instance, the electrical signal may comprise (about) 10 cathodic phases and anodic phases and subsequently a (single) gap, optionally followed by again (about) 10 cathodic phases and anodic phases.

In further embodiments, the gap phases may have gap durations $T_G$ individually selected from the range of 1 - 120 $\mu$s, especially from the range of 1 - 90 $\mu$s, such as from the range of 1 - 60 $\mu$s, especially from the range of 1 - 50 $\mu$s, such as from the range of 1 - 30 $\mu$s or the range from 1-8 $\mu$s. In further embodiments, each gap phase may have a gap duration $T_G$ of at least 1 $\mu$s, especially at least 3 $\mu$s, such as at least 4 $\mu$s, especially at least 5 $\mu$s, such as at least 8 $\mu$s, especially at least 15 $\mu$s. In further embodiments, each gap phase may have a gap duration $T_G$ of at most 120 $\mu$s, such as at most 90 $\mu$s, especially at most 60 $\mu$s, such as at most 30 $\mu$s. In further embodiments, each gap phase may have a gap duration $T_G$ of at most 20 $\mu$s, such as at most 12 $\mu$s, especially at most 8 $\mu$s.

In particular, in embodiments, the cathodic phases, anodic phases, and the interspersed gap phases may all have (essentially) the same duration (or "pulse widths"), especially during the therapeutic period(s). For instance, in embodiments, the cathodic phases, anodic phases, and the interspersed gap phases may have durations of 15-25 $\mu$s, such as of about 20 $\mu$s. Such pulse widths, particularly together with rectangular pulse shapes, may be particularly suitable for effective blocking at low onset excitation.

In a further aspect, the invention provides a method for inhibiting conduction of an action potential in (an axon of) a nerve. In particular, the method may comprise providing an electrical signal to the nerve via one or more electrodes, especially via the electrode(s) of the system of the invention. In embodiments, the electrical signal may have a frequency $F_E$ selected from the range of 5 - 100 kHz, such as form the range of 5 - 50 kHz, especially from the range of 5-25 kHz. In further embodiments, the electrical signal may have a (peak) amplitude $A_E$ selected from the range of 1 - 20 mA, such as from the range of 2 - 10 mA. In further embodiments, the electrical signal may comprise a plurality of phases, wherein the plurality of phases comprises cathodic phases and anodic phases, especially alternating cathodic phases and anodic phases, and especially wherein each phase has a phase width $T_P$ individually selected from the range of 1 - 200 $\mu$s, such as from the range of 5 - 60 $\mu$s. In further embodiments, the electrical signal may, especially during a therapeutic period, have a charge per time CPT selected from the range of 0.25 - 7.0 mC/s, such as from the range of 1.0 - 7.0 mC/s, or such as from the range of 0.7 - 4.0 mC/s, wherein CPT = $F_E * A_E * T_P$.

In specific embodiments, the invention may provide a method for inhibiting conduction of an action potential in (an axon of) a nerve, wherein: the method comprises providing an electrical signal to the nerve via one or more electrodes wherein: the electrical signal has a frequency $F_E$ selected from the range of 5 - 100 kHz; the electrical signal has an amplitude $A_E$ selected from the range of 1 - 20 mA; the electrical signal comprises a plurality of phases, wherein the plurality of phases comprises alternating cathodic phases and anodic phases, wherein each phase has a duration $T_P$ individually selected from the range of 1 - 200 $\mu$s; and the electrical signal has a charge per time CPT selected from the range of 0.25 - 7.0 mC/s, wherein CPT = $F_E * A_E * T_P$. In further embodiments, the invention may provide a method for inhibiting conduction of an action potential in (an axon of) a nerve, wherein: the method comprises providing an electrical signal to the nerve via one or more electrodes wherein: the electrical signal has a frequency $F_E$ selected from the range of 5 - 50 kHz; the electrical signal has an amplitude $A_E$ selected from the range of 2 - 10 mA; the electrical signal comprises a plurality of phases, wherein the plurality of phases comprises alternating cathodic phases and anodic phases, wherein each phase has a duration $T_P$ individually selected from the range of 5 - 60 $\mu$s; and the electrical signal has a charge per time CPT selected from the range of 1.0 - 7.0 mC/s, wherein CPT = $F_E*A_E*T_P$.

The method of the invention may, analogously to the system (see above), facilitate efficiently and selectively blocking action potential conduction in a nerve (fiber), with limited onset and offset effects.

In embodiments, the method comprises providing an electrical signal to the nerve via one or more electrodes, especially via a single electrode, or especially via two electrodes, or especially via three electrodes, or especially via four or more electrodes. In embodiments, the method may comprise providing the electrical signal using the (electrodes of) the arrangement as described above.

In further embodiments, the electrical signal may comprise a therapeutic period (see above), especially a plurality of therapeutic periods.

In further embodiments, the electrical signal may, especially during the therapeutic period, have a frequency $F_E$, wherein the frequency $F_E$ is selected from the range of 2 - 100 kHz, such as from the range of 3 - 50 kHz, especially from the range of 5 - 25 kHz, such as from the range of 10 - 25 kHz, especially from the range of 10 - 20 kHz. In further embodiments, the frequency $F_E$ may be selected from the range of 5 - 100 kHz, such as from the range of 8 - 50 kHz, especially from the range of 10 - 35 kHz, such as from the range of 20 - 33 kHz. In further embodiments, the frequency $F_E$ may be at least 2 kHz, such as at least 3 kHz, especially at least 5 kHz, such as at least 7 kHz, especially at least 8 kHz, such as at least 10 kHz. In further embodiments, the frequency $F_E$ may be at least 15 kHz, such as at least 20 kHz. In further embodiments, the frequency $F_E$ may be at most 100 kHz, such as at most 70 kHz, especially at most 50 kHz, such as at most 35 kHz, especially at most 33 kHz. In particular, frequencies $F_E$ of 20 kHz or higher may be particularly suitable for limiting, especially preventing, onset effects (see below).

In further embodiments, the electrical signal may, especially during a therapeutic period, have an (absolute) amplitude $A_E$, wherein the amplitude $A_E$ is selected from the range of 1 - 20 mA, especially in the range of 2 - 15 mA, such as from the range of 2 - 10 mA, especially from the range of 3 - 5 mA, or especially from the range of 5 - 8 mA. In further embodiments, the amplitude $A_E$ may be selected from the range of 2.5 - 12 mA, especially form the range of 2 - 6 mA, such as from the range of 4 - 6 mA. In further embodiments, the amplitude $A_E$ may be at least 1 mA, such as at least 2 mA, especially at least 2.5 mA, such as at least 3 mA, especially at least 5 mA. In further embodiments, the amplitude $A_E$ may be at most 20 mA, such as at most 15 mA, like at most 12 mA, especially at most 10 mA, such as at most 8 mA, especially at most 5 mA.

In further embodiments, the electrical signal may, especially during the therapeutic period, comprise a plurality of phases. The plurality of phases may comprise cathodic phases and anodic phases. In embodiments, the cathodic phases and the anodic phases may be alternatingly arranged in time, i.e.: Cathodic-Anodic-C-A-C-A-C-A-.... Hence, the plurality of phases may comprise alternating cathodic phases and anodic phases. In further embodiments, the cathodic phases may be arranged in time such that two successive cathodic phases are followed by two successive anodic phases and *vice versa,* i.e.,: Cathodic-Cathodic-Anodic-Anodic-C-C-A-A-C-C-A-A-....

In embodiments, to prevent charge accumulation, a series of successive cathodic and anodic phases may, together, be (essentially) charge balanced. In particular, in embodiments, each cathodic phase may be charged balanced with a preceding and/or subsequent anodic phase. A series of phases may herein be considered "charge balanced" when there is effectively no (net) accumulation of charge as a result of the phases.

Hence, the method may comprise providing an electrical signal wherein the cathodic phases and the anodic phases are (together) charge balanced.

In embodiments, the method may comprise providing the electrical signal such that an overall potential remains below 150 mV, such as below 100 mV, especially below 50 mV, i.e., the operational parameters of the electrical signal may be chosen such that an overall potential remains below 150 mV, such as below 100 mV, especially below 50 mV.

In embodiments, the method may comprise mitigating a DC offset (to address charge accumulation) via one or more of ground shunting, shunting a stimulation electrode to a counter electrode, modulating an anodic amplitude, modulating an anodic pulse width, and/or the insertion of an extra counter pulse.

In embodiments, each phase (of the plurality of phases) may, especially during the therapeutic period, have a pulse width $T_P$ (or "duration $T_P$") individually selected from the range of 3 - 120 μs, especially from the range of 4 - 90 μs, such as from the range of 5 - 60 μs, especially from the range of 10 - 50 μs, such as from the range of 15 - 50 μs. In further embodiments, each phase (of the plurality of phases) may, especially during the therapeutic period, have a pulse width $T_P$ (or "duration $T_P$") individually selected from the range of 1 - 200 μs, such as from the range of 2 - 100 μs, especially from the range of 3 - 80 μs. In further embodiments, each phase (of the plurality of phases) may have a pulse width $T_P$ of at least 1 μs, such as at least 2 μs, especially at least 3 μs, such as at least 4 μs, especially at least 5 μs, such as at least 10 μs, especially at least 15 μs. In further embodiments, each phase (of the plurality of phases) may have a pulse width $T_P$ of at most 120 μs, such as at most 90 μs, especially at most 60 μs, such as at most 50 μs, especially at most 30 μs.

In specific embodiments, the electrical signal may have a frequency $F_E$ selected from the range of 10 - 35 kHz, an amplitude $A_E$ selected from the range of 5 - 8 mA, and for each phase (of the plurality of phases) the duration $T_P$ may be (individually) selected from the range of 15 - 50 μs. In further embodiments the electrical signal may have a frequency $F_E$ selected from the range of 10 - 35 kHz, an amplitude $A_E$ selected from the range of 2.5 - 12 mA, and for each phase (of the plurality of phases) the duration $T_P$ may be (individually) selected from the range of 3 - 80 $\mu$s.

In further embodiments, during the therapeutic period, the electrical signal may have a charge per time CPT selected from the range of 0.25 - 7.0 mC/s, such as from the range of 1.0 - 7.0 mC/s, or such as from the range of 0.75 - 6.5 mC/s, especially from the range of 1 - 6.0 mC/s. In further embodiments, during the therapeutic period, the electrical signal may have a charge per time CPT selected from the range of 0.5 - 4.0 mC/s, such as from the range of 0.7 - 3.0 mC/s, such as from the range of 0.8 - 2.0 mC/s, especially from the range of 0.9 - 1.5 mC/s, wherein CPT = $F_E*A_E*T_P$.

In embodiments wherein the electrical signal comprises a plurality of therapeutic periods, especially for each therapeutic period may apply that CPT is (individually) selected from the range of 0.25 - 7.0 mC/s, such as from the range of 1.0 - 7.0 mC/s, or such as from the range of 0.75 - 6.5 mC/s, especially from the range of 1 - 6 mC/s. In further embodiments, for each therapeutic period may apply that CPT is (individually) selected from the range of 0.5 - 4.0 mC/s, such as from the range of 0.7 - 3.0 mC/s, such as from the range of 0.8 - 2.0 mC/s, especially from the range of 0.9 - 1.5 mC/s.

In further embodiments, during the therapeutic period, especially during each therapeutic period, CPT may be at least 0.25 mC/s, especially at least 0.5 mC/s, such as at least 0.6 mC/s, especially at least 0.7 mC/s, such as at least 0.75 mC/s. In further embodiments, during the therapeutic period, especially during each therapeutic period, CPT may be at least 0.8 mC/s, especially at least 0.9 mC/s, such as at least 1.0 mC/s, especially at least 1.2 mC/s, such as at least 1.5 mC/s. In further embodiments, during the therapeutic period, especially during each therapeutic period, CPT may be at most 7.0 mC/s, especially at most 6.5 mC/s, such as at most 6.0 mC/s, especially at most 5.0 mC/s. In further embodiments, during the therapeutic period, especially during each therapeutic period, CPT may be at most 4.0 mC/s, such as at most 3.0 mC/s, especially at most 2.5 mC/s, such as at most 2.0 mC/s, especially at most 1.5 mC/s.

In embodiments, the method may be for preventing and/or treating disease in a subj ect.

In further embodiments, the disease may comprise a respiratory disease selected from the group comprising chronic obstructive pulmonary disease (COPD), asthma, sleep apnea, asthma-COPD overlap syndrome, cystic fibrosis, interstitial lung disease, lung fibrosis, and pulmonary hypertension. Especially, in such embodiments, the (to be inhibited) action potential conduction may be related to one or more of bronchoconstriction, mucus hypersecretion and inflammation.

In further embodiments, the disease may comprise COPD.

In further embodiments, the disease may comprise a cardiac disease selected from the group comprising atrial fibrillation, symptomatic clinical bradyarrhythmia, vasovagal syncope, sinus node dysfunction, and functional atrioventricular block. In such embodiments, the (to be inhibited) action potential conduction may thus especially be related to hyperactivity (or pathological activity) in the vagus nerve.

In embodiments, the nerve comprises a vagus nerve.

In further embodiments, the method comprises applying the electrical signal to the nerve to stimulate a B-fiber in the nerve, especially in the vagus nerve.

In further embodiments, the method comprises applying the electrical signal to the nerve to stimulate an A-fiber or a C-fiber in the nerve, especially an A-fiber, or especially a C-fiber.

In embodiments, the electrical signal may have a signal duration $T_E$, wherein $T_E$ is selected from the range of 1 s - 24 h, especially from the range of 10 s - 24 h, such as from the range of 30 s - 12 h. In further embodiments, $T_E$ may be selected from the range of 5 s - 18 h, such as from the range of 10 s - 12 h. In further embodiments, $T_E$ may be selected from the range of 1 s - 1200 s, such as from the range of 5 s - 600 s, especially from the range of 10 s- 300 s,

As described above, the electrical pulse may essentially have any pulse shape. In embodiments, the method may comprise providing the electrical signal, especially the pulse and the counter pulse, with a (symmetric) rectangular pulse shape, especially a square pulse shape. In further embodiments, the method may comprise providing the electrical with a sinusoidal or triangular pulse shape.

The embodiments described herein are not limited to a single aspect of the invention. For example, an embodiment describing the method may, for example, further relate to the system, especially to an operational mode of the system, or especially to the control system. Similarly, an embodiment of the system describing an operation of the system may further relate to embodiments of the method. In particular, an embodiment of the method describing an operation (of the system) may indicate that the system may, in embodiments, be configured for and/or be suitable for the operation. Similarly, an embodiment of the system describing actions of (a stage in) an operational mode may indicate that the method may, in embodiments, comprise those actions.

Further, in embodiments, the system, especially the control system, may be configured to execute the method of the invention.

In further embodiments, the method may comprise using the system of the invention, especially applying the electrical signal (to the nerve) using the system of the invention.

BRIEF DESCRIPTION OF THE DRAWINGS

Embodiments of the invention will now be described, by way of example only, with reference to the accompanying schematic drawings in which corresponding reference symbols indicate corresponding parts, and in which: Fig. 1A-C schematically depict embodiments of the system of the invention. Fig. 2 schematically depicts a further embodiment of the system of the invention. Fig. 3 schematically depicts embodiments of the system and the method of the invention. Fig. 4A-D schematically depict experimental and simulated results for embodiments of the invention. Fig. 5 schematically depicts an embodiment of an electrical signal. The schematic drawings are not necessarily on scale.

DETAILED DESCRIPTION OF THE EMBODIMENTS

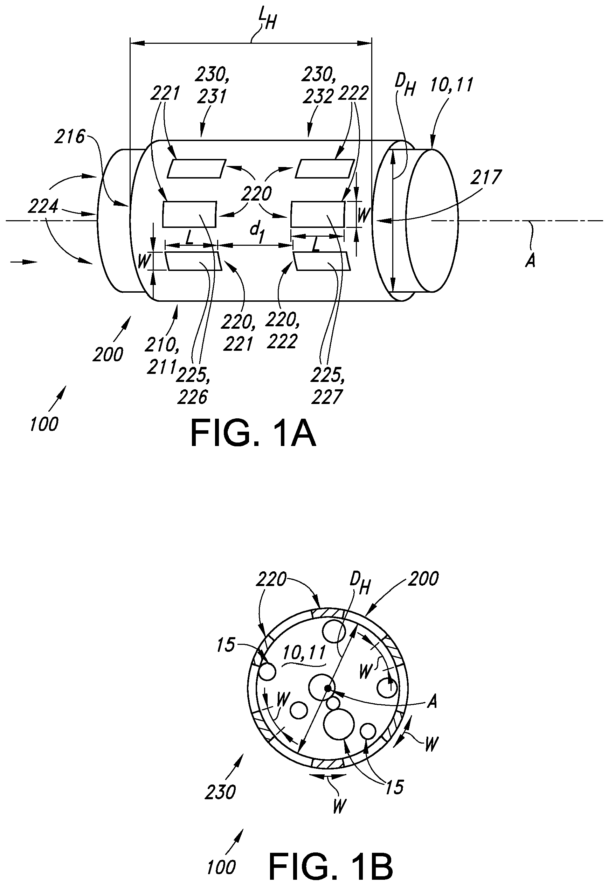

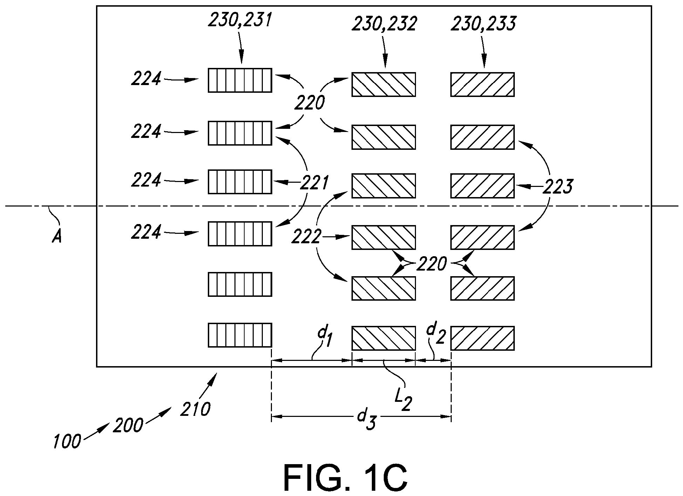

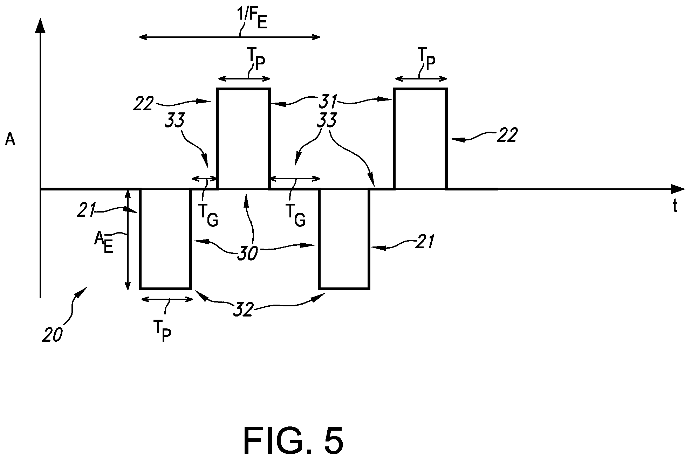

Fig. 1 schematically depicts a system for inhibiting action potential conduction in (an axon of) a nerve 10. In the depicted embodiment, the system 100 comprises an arrangement 200, especially a cuff arrangement. The arrangement 200 comprises a hosting element 210, especially a tubular hosting element 211, configured for at least partially surrounding the nerve 10, wherein the hosting element 210 comprises a plurality of electrodes 220. In embodiments, the system 100 may be configured to apply in a treatment mode of the system 100 an electrical signal 20 (to the nerve 10) via at least part of the plurality of electrodes 220. In particular, in embodiments, one or more, especially all, of the following may apply: (a) the electrical signal 20 may have a frequency $F_E$ selected from the range of 5 - 100 kHz, such as from the range of 5 - 50 kHz, especially from the range of 5 - 25 kHz; (b) the electrical signal 20 may have an (absolute) amplitude $A_E$ selected from the range of 1 - 20 mA, such as from the range of 2 - 10 mA; (c) the electrical signal 20 may comprise a plurality of phases 30 (see Fig. 5), wherein the plurality of phases 30 comprises alternating cathodic phases 31 and anodic phases 32, and wherein each phase 30 (of the plurality of phases 30) has a pulse width $T_P$ individually selected from the range of 1 - 200 $\mu$s, such as from the range of 5 - 60 $\mu$s; and (d) the electrical signal 20 comprises a therapeutic period, wherein $F_E$, $A_E$ and $T_P$ are (essentially) constant during the therapeutic period, and wherein during the therapeutic period the electrical signal 20 has a charge per time CPT selected from the range of 0.25 - 7.0 mC/s, especially from the range of 1.0 - 7.0 mC/s, or especially from the range of 0.7 - 4 mC/s, such as from the range of 2 - 3.5 mC/s, or such as from the range of 1.5 - 2.5 mC/s, or especially from the range of 0.8 - 2.0 mC/s, wherein CPT = $F_E*A_E*T_P$.

As described above, the hosting element 210, especially the tubular hosting element 211, may have a longitudinal axis A, especially wherein, during use, the longitudinal axis A is aligned with a dimension of elongation of the nerve. In particular, in the depicted embodiment, the hosting element 210 comprises a tubular hosting element 211 having an elongated tubular shape with a first opening 216 and a second opening 217 at opposite ends, wherein the nerve enters the tubular hosting element through a first opening 216 and exits the tubular hosting element through the second opening 217.

In the depicted embodiment, the (tubular) hosting element 210 has an (inner) diameter $D_H$ and a length $L_H$, wherein the diameter $D_H$ is defined perpendicular to the longitudinal axis A and wherein the length is defined along the longitudinal axis A. The diameter $D_H$ may especially be an average equivalent circular diameter of the hosting element 210.

Further, in the depicted embodiment, along the longitudinal axis A of the hosting element 210 each electrode 220 has a length (L) individually selected from the range of 0.1*D - 2*D mm, especially from the range of 0. 1*$D_H$ - 0.5*$D_H$. Further, in the depicted embodiment, circumferentially around the longitudinal axis A each electrode 220 has a width W individually selected from the range of 0.1*$D_H$ - 2*$D_H$ mm, especially from the range of 0.1*$D_H$ - 0.75*$D_H$ mm.

Fig. 1A schematically depicts an embodiment wherein the electrodes 220 are arranged in a plurality of electrode arrays 230. In particular, in the depicted embodiment, the electrodes 220 comprise first electrodes 221 and second electrodes 222, wherein the first electrodes 221 are circumferentially arranged in the tubular hosting element in a first electrode array 231, and wherein the second electrodes 222 are circumferentially arranged in the tubular hosting element in a second electrode array 232. Further, (first electrodes 221 of) the first electrode array 231 and (second electrodes 222 of) the second electrode array 232 are separated by a first edge-to-edge distance $d_1$ along the longitudinal axis A. In embodiments, the first edge-to-edge distance $d_1$ may be selected from the range of 0.2 - 5 mm, especially from the range of 0.5 - 3 mm, such as from the range of 0.5 - 2 mm.

The electrodes 220 of the first electrode array 231 and the second electrode array 231 may, as depicted in Fig. 1A, be longitudinally aligned. In particular, for each first electrode 221 (of a first subset of the first electrodes 221) may apply that: the first electrode 221 is longitudinally aligned with a (respective) second electrode 222 (of a second subset of the second electrodes 222). In such embodiments, (contact) surface areas 225 of the first electrode 221 and of the second electrode 222 may differ by less than 5% (relative to the largest surface area of the first and the second electrode).

In particular, Fig. 1A schematically depicts an embodiment wherein the system 100 comprises a thin-film cuff arrangement with cylindrical shape that includes a multitude of electrode contacts (each forming an interface between a high charge-injection electrode material and the nerve tissue) mainly but not exclusively on the inner side of the cuff arrangement, arranged in at least two circumferentially-spread arrays of 4 to 12, especially 6, contacts each, such that each contact in an array has at least one longitudinally aligned counterpart contact, thereby providing a bipolar configuration for stimulation.

Hence, in embodiments, the hosting element 210 may comprise a plurality of sets 224 of longitudinally aligned electrodes 220, wherein for each set 224 applies that: the set 224 comprises a first electrode 221 and a second electrode 222, wherein the first electrode 221 has a first (contact) surface area 226, and wherein the second electrode 222 has a second (contact) surface area 227, wherein the first surface area 226 and the second surface area 227 differ by less than 5%, such as less than 3%, especially less than 1%, including 0%.

Especially, as in the depicted embodiment, longitudinally aligned electrodes 220 may have equal lengths L and widths W.

In further embodiments, one or more of the electrodes 220 may comprise ring electrodes. For instance, instead of the first electrode array 230, a ring electrode may be used.

Fig. 1B schematically depicts a cross-sectional view of the system 100 depicted in Fig. 1A. In particular, Fig. 1B schematically depicts an electrode array 230 comprising six electrodes 220, wherein the electrodes are circumferentially arranged in the (tubular) hosting element 210. Further, as the (tubular) hosting element 210 is arranged around the nerve 10, Fig. 1B further schematically depicts the electrodes 220 of the electrode array 230 circumferentially arranged around the nerve 10, especially around the vagus nerve 11.

Fig. 1B further schematically depicts nerve fibers 15 in the nerve 10. As depicted, nerve fibers 15 may be centrally or peripherally located in the nerve 10. For providing a signal to a peripherally arranged nerve fiber 15, electrodes in close proximity to the nerve fiber 15 may be most suitable to selectively target the corresponding nerve fiber 15. Hence, it may be beneficial to have electrodes circumferentially arranged around the nerve for selective targeting.

Further, when two electrodes that are spaced relatively close together (along the longitudinal axis) are used for stimulation, the electrical signal may pass through the nerve relatively close to a surface of the nerve, and may thus be most suitable for targeting laterally arranged (or "superficial") nerve fibers 15. In contrast, when two electrodes that are spaced relatively far apart (along the longitudinal axis) are used for stimulation, the electrical signal may pass through the nerve close to the center of the nerve, and may thus be most suitable for targeting medially arranged (or "deeper") nerve fibers 15. Further, also electrode length (along the longitudinal axis) may influence the ability to target laterally vs. medially arranged nerve fibers. In particular, smaller electrodes may be more suitable for targeting peripherally arranged nerve fibers, whereas longer electrodes may be more suitable for targeting centrally arranged nerve fibers.

Hence, it may be beneficial to provide an arrangement with three or more electrode arrays to provide additional flexibility in selecting the distances between the electrodes used for providing the electrical signal.

Fig. 1C schematically depicts an embodiment of the system, especially of the arrangement, wherein the hosting element comprises a first electrode array 231, a second electrode array 232 and a third electrode array 233. In the depicted embodiment, the hosting element 210 may, for visualization purposes especially be depicted flat (or "unrolled").

In particular, in the depicted embodiment, the first electrode array 231 comprises first electrodes 221, the second electrode array 232 comprises second electrodes 222, and the third electrode array 233 comprises third electrodes 223. In particular, in the depicted embodiment, the electrodes 220 of the three arrays 230 may be longitudinally aligned along the longitudinal axis. In particular, the hosting element 210 comprises sets 224 of longitudinally aligned electrodes, wherein each set 224 comprises a first electrode 221, a second electrode 222 and a third electrode 223.

The (first electrodes 221 of the) first electrode array 231 and (second electrodes 222 of) the second electrode array 232 are separated by a first edge-to-edge distance $d_1$ along the longitudinal axis A. In embodiments, the first edge-to-edge distance $d_1$ may be selected from the range of 0.2 - 5 mm, especially from the range of 0.5 - 3 mm, such as from the range of 0.5 - 2 mm.

Similarly, the (second electrodes 222 of the) second electrode array 232 and the (third electrodes 223 of the) second electrode array 233 are separated by a second edge-to-edge distance $d_2$ along the longitudinal axis A. In embodiments, the second edge-to-edge distance $d_2$ may be selected from the range of 0.2 - 5 mm, especially from the range of 0.5 - 3 mm. As depicted in Fig. 1C, the first distance $d_1$ and the second distance $d_2$ may especially differ.

As depicted in Fig. 1C, the (first electrodes 221 of the) first electrode array 231 and the (third electrodes 223 of the) third electrode array 233 may be separated by a third edge-to-edge distance $d_3$ along the longitudinal axis A, wherein $d_3 = d_1 + d_2 + L_2$, wherein $L_2$ is the length of a second electrode 222 (along the longitudinal axis A).

Further, in the depicted embodiment, for each first electrode 221 (of a first subset of the first electrodes 221) applies that: the first electrode is longitudinally aligned with a (respective) third electrode 223 (of a third subset of the third electrodes 223), wherein (contact) surface areas of the first electrode 221 and of the third electrode 223 differ by less than 5%, such as by less than 3%, especially by less than 1%. In particular, in the depicted embodiment, the (contact) surface areas of the first, second and third electrodes may be essentially the same.

Fig. 1C further schematically depicts a first electrode array 231 comprising $n_1$ first electrodes 221, and second electrode array 232 comprising $n_2$ second electrodes, and a third electrode array 233 comprising $n_3$ third electrodes, wherein each of $n_1$, $n_2$ and $n_3$ are selected from the range of 3 - 12, especially from the range of 4 - 10. In particular, in the depicted embodiment, $n_1 = n_2 = n_3 = 6$.

In further embodiments, the edge-to-edge distance between electrodes 220 of the same electrode array 230 may be selected from the range of 0.1*W - 5*W, with W as the electrode width W. For instance, in embodiments, edge-to-edge distances between first electrodes 221 in the first electrode array 231 may be (individually) selected from the range of 0.1*W - 5*W, such as from the range of 0.5*W - 2*W. In further embodiments, edge-to-edge distances between second electrodes 222 in the second electrode array 232 may be (individually) selected from the range of 0.1*W - 5*W, such as from the range of 0.5*W - 2*W. In further embodiments, edge-to-edge distances between third electrodes 223 in the third electrode array 233 may be (individually) selected from the range of 0.1*W - 5*W, such as from the range of 0.5*W - 2*W.

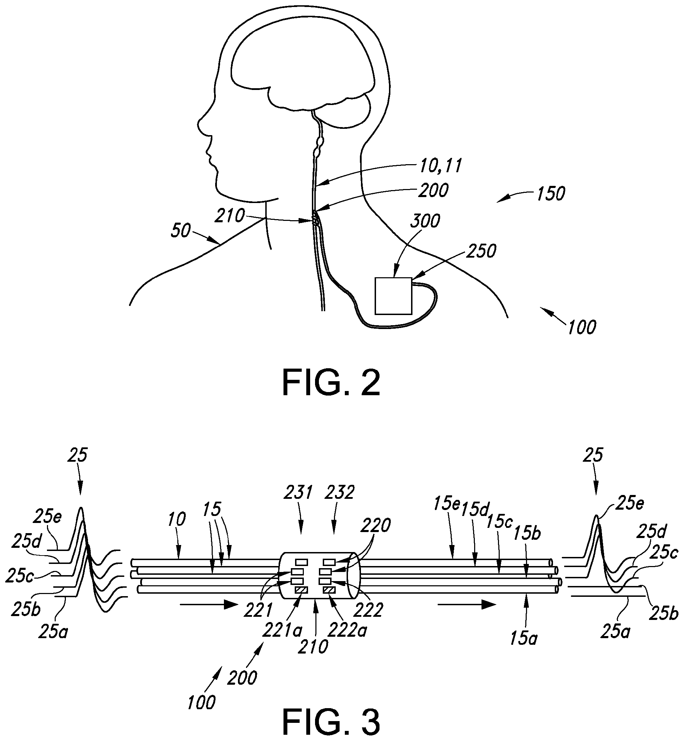

Fig. 2 schematically depicts an embodiment of the system 100, wherein the system comprises an (implantable) device 150, wherein the device 150 is implanted in a subject 50, especially at the neck of the subject 50. Hence, the device 150 may be configured to be implanted in the subject 50, especially at the neck of the subject 50. In particular, in the depicted embodiment, the device 150 comprises the arrangement 200, wherein the arrangement 200 comprises the hosting element 210, wherein the hosting element hosts the nerve 10, here especially the vagus nerve 11, of the subject.

In such embodiments, the device 150, especially the arrangement, 200 may comprise a biocompatible material, especially at least at external surfaces of the device 150. Further, in embodiments, the arrangement 200 may comprise a biocompatible carrier substrate, wherein the biocompatible carrier substrate comprises openings, wherein the electrodes 220 are configured to contact the nerve 10 through the openings.

In the depicted embodiment, the device 150 further comprises an (implantable) pulse generator 250. The pulse generator 250 may especially be configured to provide the electrical pulse to the nerve via (at least part of) the electrodes 220 of the arrangement 200.

In the depicted embodiment, the device 150 further comprises a control system 300. The control system 300 may especially be configured to control one or more of the pulse generator 250 and the electrodes 220.

Fig. 3 schematically depicts a further embodiment of the system 100 of the invention. In Fig. 3, the hosting element 210 is depicted hosting the nerve 10, wherein the nerve 10 comprises five nerve fibers 15: 15a, 15b, 15c, 15d and 15e. For visualization purposes, the fibers are all depicted as being configured to conduct action potentials in the same direction, e.g., all are efferent directed fibers. In practice, a nerve may comprise both afferent and efferent fibers.

Fig. 3 further schematically depicts action potentials 25 travelling along the nerve fibers 15 of the nerve 10 from the left to the right. Specifically, on the left side of the nerve five action potentials 25 upstream of the hosting element 210 are depicted and on the right side of the nerve five action potentials 25 downstream of the hosting element 210 are depicted. For explanatory purposes, each of these action potentials 25 is associated with a respective nerve fiber 15, i.e.,: action potential 25a travels along nerve fiber 15a; action potential 25b travels along nerve fiber 15b; action potential 25c travels along nerve fiber 15c; action potential 25d travels along nerve fiber 15d; and action potential 25e travels along nerve fiber 15e. In the depicted embodiment, an electrical signal is applied to the nerve 10 via a selected first electrode 221 and a selected second electrode 222a, which are arranged in proximity to a first fiber 15a and a second fiber 15b. As schematically depicted on the right side, i.e., downstream, of the hosting element 210, the first action potential 25a and the second action potential 25b have been effectively blocked, while the third-fifth action potentials 25c, 25d, 25e continue (essentially) unimpeded.

The terms "upstream" and "downstream" herein relate to an arrangement of items or features relative to the propagation of an action potential in a nerve relative to a source of the action potential, wherein relative to a first position along the nerve, a second position along the closer to the source is "upstream", and a third position along the nerve further away from the source is "downstream".

Fig. 3 further schematically depicts an embodiment of the method for inhibiting conduction of an action potential 25 in (a nerve fiber 15 of) a nerve 10. In the depicted embodiment, the method comprises providing an electrical signal 20 to the nerve 10 via one or more electrodes 220, here especially via a selected first electrode 221a and a selected second electrode 222a, wherein the selected first electrode 221a and the selected second electrode 222a are longitudinally aligned along the nerve. The electrical signal may have parameters suitable for blocking the conduction of an action potential 25 in the nerve 10. In particular, the electrical signal may, especially during a therapeutic period, have: (a) a frequency $F_E$ selected from the range of 5 - 100 kHz, such as from the range of 5 - 50 kHz, especially from the range of 5-25 kHz; (b) a (peak) amplitude $A_E$ selected from the range of 1 - 20 mA, such as from the range of 2 - 10 mA; (c) a plurality of phases 30, wherein the plurality of phases 30 comprises alternating cathodic phases 31 and anodic phases 32, especially wherein each phase has a duration $T_P$ individually selected from the range of 1 - 200 $\mu$s, such as from the range of 5 - 60 $\mu$s, and a charge per time CPT selected from the range of 0.25 - 7.0 mC/s, especially from the range of 1.0 - 7.0 mC/s, or especially from the range of 0.7 - 4.0 mC/s, such as from the range of 0.8 - 2.0 mC/s, wherein CPT = $F_E*A_E*T_P$.

For instance, in embodiments, the method may comprise applying the electrical signal 20 to the nerve 10 to stimulate a B-fiber in the nerve 10.

## Experiments

Experiments were performed in sheep as a large animal model.

The experiments were performed using four different arrangements (or "cuff designs". The cuff designs varied in electrode width, electrode length, number of electrodes per electrode array, number of electrode arrays, edge-to-edge distance $d_1$ between electrodes, and geometrical electrode contact area (for cathode and anode), as summarized in table X below:

| Design name | RevD1 | RevD2 | RevD3 | RevD4 |
|---|---|---|---|---|
| Electrode width W [mm] | 1.38 | 0.43 | 0.805 | 0.43 |
| Electrode length L [mm] | 0.43 | 0.92 | 0.43 | 0.43 |
| Number of arrays [number] | 4 | 2 | 2 | 2 |
| Electrodes per array [number] | 3 | 6 | 6 | 6 |
| Edge-to-edge distance $d_1$ [mm] | 0.365 - 1.965 | 0.65 | 0.8 | 0.8 |
| Geometrical electrode contact area [$mm^2$] | 0.182 | 0.099 | 0.099 | 0.050 |

## Experiments

Unless specified otherwise, Experiments 1 and 2 as described below were executed using the following procedures.

All experiments in sheep were performed by DVMs (Doctor of Veterinary Medicine) after obtaining regulatory and ethical clearance. Studies were conducted in compliance with ISO 10993-2 and applicable standards of the Clinical Research Organization (CRO). Studies were performed between 2022 and 2024. Female sheep from the Ile-de-France breed with a weight between 45 and 65 kg were obtained from local breeders. The health status of the sheep was monitored by the CRO. After acclimation, sheep were premedicated with morphine (0.2 mg/kg intramuscularly) and midazolam (0.5 mg/kg intramuscularly). Anesthesia was induced with propofol 2-4 mg/kg intravenously (IV). Study animals were then intubated with an appropriately sized endotracheal tube (between 7-9 mm diameter) and maintained with a constant rate infusion of propofol 5-30 mg/kg/h intravenously and repeated boli of midazolam 0.5 mg/kg intravenously. Respiratory rates were set at 10-20 cycles /min, and a tidal volume of 10 ml/kg with 100% fractional $pO_2$. Respiratory rates were adapted to end-tidal $CO_2$ with a target of approximately 40 cm $H_2O$. Fluid replacement was done with lactated Ringer (5 to 20 mL/kg/h). The following physiological parameters were obtained: ECG (1 or 3-lead), body temperature and local temperature at the left implantation site, systemic and pulmonary artery pressure, pulse oximetry, end-tidal $pCO_2$, airway resistance, compliance, EMGs of the cricothyroideal and cricopoharyngeal muscles. Parameters were acquired by anesthesia monitors and/or the ADI powerlab system (ADInstruments Limited, Oxford, UK).

The left vagus nerve was exposed to a maximum length and freed from any adhering tissue. An access to the right vagus nerve was also performed, and the nerve entwined by a ligature for later recovery. EMG electrodes were placed, and 2 to 4 cuff electrodes placed around the nerve. Electrodes were connected to either a Subject interface stimulator (Controlled by a RZ2, Tucker-Davis Technologies, Alachua, FL, USA) or a Model 3820 stimulator (Controlled by a Model 3800 Multichannel Stimulator, A-M systems, Sequim, Washington, USA). Stimulation protocols were steered by proprietary software written in Matlab R2021b (The MathWorks Inc., Natick, USA). $CO_2$-exposures for induction of bronchoconstriction were done with inspirational $CO_2$ fractions of 4-10% admixed to pure oxygen. Several multichannel electrode cuffs were placed around the left cervical vagus nerve in anesthetized and ventilated adult female sheep. Electrocardiogram was recorded via two laterally placed needle electrodes on the thorax of the animal against one ground electrode placed on the right leg of the animal. Systemic blood pressure was collected via a pressure catheter (SPR-350S Mikro-Tip®, Millar Houston, Texas, USA) placed in the abdominal aorta via the femoral artery. Heart rate was calculated from the systemic blood pressure and averaged for 3 cycles. Application of active stimulation and blocking traces was simultaneously collected from the signal generator. Generally, bipolar stimulation pulses were current controlled and generated.

Experiment 1 - blocking of action potential conduction in cardiac fibers in sheep

Bradycardia was induced by the most cranial electrode and the electrode locations around the vagus nerve having the strongest effect on bradycardia were identified. Identification of the electrode contact pair which induces the strongest effects of bradycardia was performed by stimulation of each electrode contact pair with equal test pulses. The electrode contact pair inducing the strongest bradycardia, as evaluated by the relative change in heart rate, is expected to have the most effect on the bradycardia-inducing nerve fibers. HF blocks were elicited in the caudally located electrodes to block the induced efferent cardiac B fiber signals.

For assessing importance of block parameters for cardiac B fiber block data from 19 experiments performed on sheep were analyzed by regression models. Both the relative efficacy of blocking efferent B-fibers innervating the heart as well as the (unintended) so-called onset excitation effects that can occur at the start of the high-frequency stimulation were analyzed. Onset excitation effects were observed in $A\alpha$ fibers as evidenced from muscle action potentials detected by electromyography (EMG) from cricothyroideal and cricopoharyngeal muscles, and in B-fibers as evidenced by bradycardia by electrocardiogram (ECG) and increases in airway resistance ($R_{AW}$) (pulmonary B fibers). $R_{AW}$ was determined during ventilation from the P-V relationship and directly imported from the ventilator (Dräger Evita v600) after subtracting system resistance (of the tubing and the endotracheal tube) so that $R_{AW}$ as reported here refers to airway resistance within the study subject.

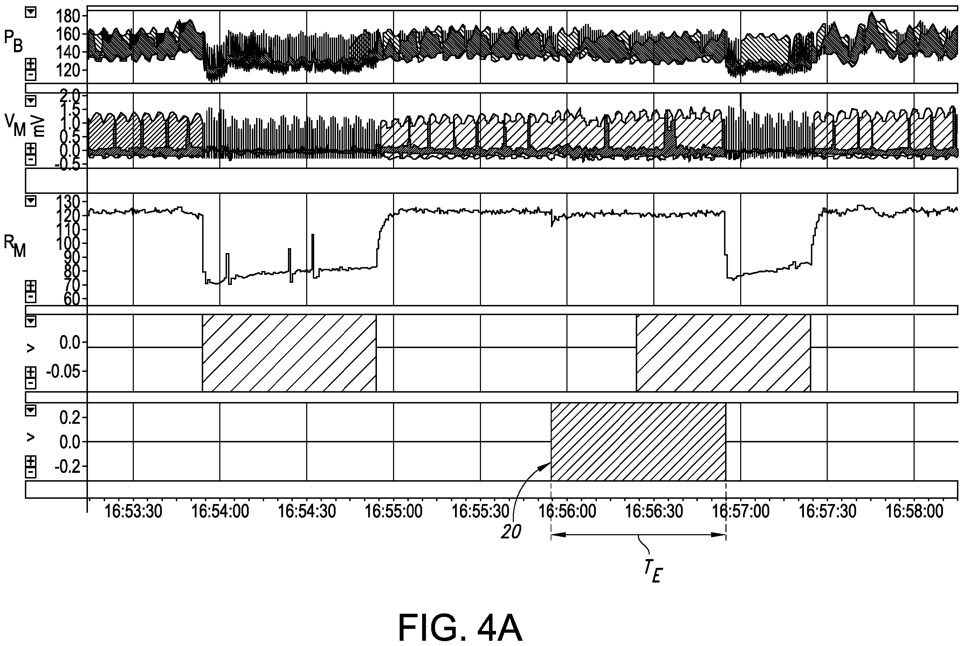

Representative effects of the induced bradycardia and the effect of the electrical signal 20 are depicted in Fig. 4A. Specifically, Fig. 4A depicts physiological recordings during pure stimulation and during a simultaneous application of the electrical signal at a more caudal location of the stimulated efferent cardiac B-fibers. Shown are from top to bottom row: (a) a systemic blood pressure $P_B$ recording (in mmHg), (b) an electrocardiogram (ECG) showing observed electrical activity $V_H$ in a heart muscle in mV, (c) the heart rate Ruin beats per minute (BPM) calculated from $P_B$ curves, (d) application of stimulation signals inducing bradycardia, and (e) application of electrical signal 20 for blocking. As may be observed in Fig. 4A, the heart rate may be substantially reduced due to the pure application of the bradycardia-inducing stimulation signal (left stimulation), may have a slight initial decrease due to application of the electrical signal 20, and may, during the application of the electrical signal 20, be essentially unaffected by the bradycardia-inducing stimulation signal (right stimulation). Further, the effect of the stimulation remains absent only until the electrical signal is turned off, i.e., the invention provides precise temporal control and complete reversibility after a few seconds over the blocking of the action potential conduction, here related to the induced bradycardia.

In the depicted embodiment, the electrical signal 20 has a signal duration $T_E$, wherein $T_E$ is selected from the range of 10 s - 12 h. In particular, in the depicted embodiment, $T_E$ is about 60 s.

Hence, in embodiments, the nerve 10 may comprise a vagus nerve 11, and the treatment mode may comprise applying the electrical signal 20 to at least one B-fiber in the nerve 10.

Block effects were quantified on the degree of reverting stimulation-induced bradycardia; complete block was inferred from a complete inhibition of drops in heartrate. Generally, block was applied (about) 30 s before and simultaneously to the bradycardia-inducing stimulation, and typically terminated (about) 30 s before the end of the bradycardia-inducing stimulation. The parameters for the bradycardia-inducing stimulation were selected to cause a heartrate drop of about 20 bpm. Block onset effects were quantified based on the degree of heartrate drop occurring at the beginning of the block. These drops in heartrate could be caused by sinus bradycardia, SA blocks, or AV blocks with escape rhythms as judged from examination of continuously measured ECGs and are expected physiological results of vagal overactivity.

For analyses, the "standard least squares" in the "fit model" platform in JMP 17.0 (SAS Institute, Cory, USA) was used. The linear regression model had the following target factors (or "dependent variables"): block effect and onset effect. The regression model further had the following x factors (or "independent variables"): (a) numerical: charge per time (CPT), frequency $F_E$, edge-to-edge distance $d_1$, and electrode width W (as ordinal factor) and electrode length L.

CPT was modelled as spline with 5 knot points, frequency was log transformed, and edge-to-edge distance $d_1$ was squared as these transformations provided the best overall models. The used data included 563 instances for both target factors, i.e., N=563 for the model. The model had adjusted $R^2$ values between 0.10 and 0.21 for the different targets. The effect tests for the blocking effect are shown in table 2:

**Effect test for Block effect $B_E$**

| Source | Nparm | DF | Sum of Squares | F Ratio | p-value |
|---|---|---|---|---|---|
| CPT&Knotted | 4 | 4 | 67040.630 | 13.3382 | <.0001* |
| Log($F_E$) | 1 | 1 | 21395.927 | 17.0274 | <.0001* |
| Square(edge-to-edge distance d1) | 1 | 1 | 2278.714 | 1.8135 | 0.1786 |
| electrode_width | 2 | 2 | 8828.621 | 3.5130 | 0.0305* |
| electrode_length | 1 | 1 | 7642.979 | 6.0825 | 0.0140* |

Nparm refers to the number of parameters associated with the 'Source'; DF refers to the Degrees of Freedom; the F ratio refers to the ratio of between group variance to within group variance. The asterisk indicates statistical significance of the parameter on the indicated effect. Hence, charge per time, the frequency and electrode dimensions (width and length) were found to be statistically significant in explaining part of the variance observed in the blocking effect.

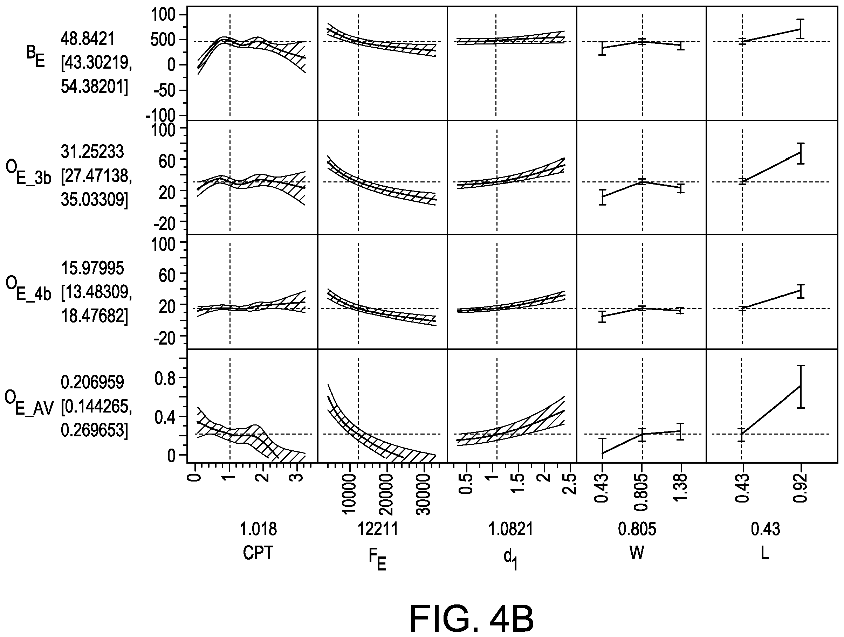

Fig. 4B schematically depicts a representative "profiler view" for the different x factors and their effect on the target factors, i.e., a representative visualization of the model predictions of the target factor for different x factor values.

In particular, Fig. 4B depicts how the block effect $B_E$ (in % signal block) and three types of onset effect $O_E$ (in % signal increase or % occurrence; see below) would vary with changing values of the x factors: charge per time CPT (in mC/s), frequency $F_E$ (in kHz), (first) edge-to-edge distance di, electrode width W and electrode length L. For each of the x factors, a specific value is chosen as a reference values; these values are indicated by the hyphenated lines in the graphs and the values indicated below the tick values of the graphs. The first row shows the block effect $B_E$, while the remaining 3 show different types of cardiac onset effects. Onset effect $O_{E,3b}$ is a quantified comparison of the change between the mean of the pre-block heartrate with the minimum heartrate within the first 15s of the block while onset effect $O_{E,4b}$ indicates the change between mean of pre-block heartrate with the mean of the heartrate within 15s of the block. While $O_{E,3b}$ accentuates the maximal drop in HR, $O_{E,4b}$ accentuates more the overall duration of the onset effect. Onset effect $O_{E,AV}$ shows occurrence of atrioventricular blocks (AV blocks), as judged by absence of a QRS complex after a P wave. AV blocks may be considered a particularly severe cardiac onset effect in contrast to milder sinus bradycardia. For all of these, higher frequency $F_E$ diminishes the occurrence of cardiac onset effects. Notably, AV blocks virtually disappear (< 0.01 fraction of instances) at frequencies of 20 kHz or higher. Hence, frequencies $F_E$ of 20 kHz or over may be strongly preferred for treatment.

CPT: As may be observed in the profiler view of Fig. 4B, CPT appears to be the most contributory factor for the efficacy of the block effect, with a steep rise of the block efficacy up to about 0.8 mC/s, and being relatively stable between about 0.8 and about 2 mC/s.

The effect tests for the onset effects are shown in tables 3-5:

**Effect test for onset effect $O_{E\_3b}$**

| Source | Nparm | DF | Sum of Squares | F Ratio | p-value |
|---|---|---|---|---|---|
| CPT&Knotted | 4 | 4 | 3618.214 | 1.5455 | 0.1876 |
| Log($F_E$) | 1 | 1 | 28061.111 | 47.9456 | <.0001* |
| Square(edge-to-edge distance) | 1 | 1 | 21940.824 | 37.4884 | <.0001* |
| electrode_width | 2 | 2 | 11306.308 | 9.6591 | <.0001* |
| electrode_length | 1 | 1 | 18745.671 | 32.0291 | <.0001* |

**Effect test for onset effect $O_{E\_4b}$**

| Source | Nparm | DF | Sum of Squares | F Ratio | p-value |
|---|---|---|---|---|---|
| CPT&Knotted | 4 | 4 | 1263.454 | 1.2375 | 0.2939 |
| Log($F_E$) | 1 | 1 | 14271.237 | 55.9108 | <.0001* |
| Square(edge-to-edge distance) | 1 | 1 | 12081.249 | 47.3310 | <.0001* |
| electrode_width | 2 | 2 | 2944.742 | 5.7683 | 0.0033* |
| electrode_length | 1 | 1 | 6801.004 | 26.6445 | <.0001* |

**Effect test for onset effect $O_{E\_AV}$**

| Source | Nparm | DF | Sum of Squares | F Ratio | p-value |
|---|---|---|---|---|---|
| CPT&Knotted | 4 | 4 | 3.4069765 | 5.2928 | 0.0003* |
| Log($F_E$) | 1 | 1 | 6.4003437 | 39.7723 | <.0001* |
| Square(edge-to-edge distance) | 1 | 1 | 3.0010527 | 18.6488 | <.0001 * |
| electrode_width | 2 | 2 | 1.0411522 | 3.2349 | 0.0401* |
| electrode_length | 1 | 1 | 3.5162084 | 21.8500 | <.0001* |

Hence, charge per time CPT, the frequency $F_E$, the edge-to-edge distance as well as the electrode dimensions are statistically significant in explaining part of the variance observed in the onset effect. In particular, the frequency $F_E$ was found to (also) be a main factor influencing the occurrence of onset effects. The best fit is a log transformation (better than linear) and the profiler view shows that up to 10 kHz the benefit of increasing the frequency is greatest, but continues with a shallower slope towards the maximally explored frequency (33 kHz). Hence, in embodiments, the electrical signal may have, especially during the therapeutic period, a frequency $F_E \geq 5$ kHz, such as $\geq 8$ kHz, especially $\geq 10$ kHz, such as $\geq 20$

kHz.

The edge-to-edge distance $d_1$ between electrodes and the electrode length L were found to be significant factors for the onset effect. With respect to the edge-to-edge distance di, distances between 0.3 and 1.2 mm appear beneficial for avoiding onset effects. A smaller electrode length L may also result in a smaller onset effect.

Hence, in embodiments the first edge-to-edge distance ($d_1$) may especially be selected from the range of 0.5 - 2 mm, such as from the range of 0.3 - 1.2 mm.

The same statistical approach was also applied solely for the cuff design RevD3 (see table 1 above; n= 326; adj R square = 0.10 - 0.15) to evaluate whether this would affect the contribution of the operational parameters and/or the preferred values thereof in view of the blocking and onset effects. No substantial changes were observed, i.e., the observations discussed above regarding the influence of frequency $F_E$ and CPT also applied (and were statistically significant) when specifically evaluating the RevD3 design.

Experiment 2 - blocking of action potential conduction in respiratory efferent B-fibers in sheep

Multielectrode cuffs were placed around the left vagus nerve in anesthetized adult female sheep. Bronchoconstriction was induced by exposure to inhalational $CO_2$ (fraction of inhaled air: 4-10%). The electrical signal was applied with amplitudes between ~2000 and 8000 $\mu$A, with a frequency of about 5-20 kHz and varying pulse widths (for the cathodic phases, anodic phases and the gap phases). Application of the electrical signal reduced the induced airway resistance by about 50% maximally, which may be expected to represent about the maximally achievable decrease in bronchoconstriction by a unilateral vagus nerve block as the stimulation affects one of both vagus nerves.

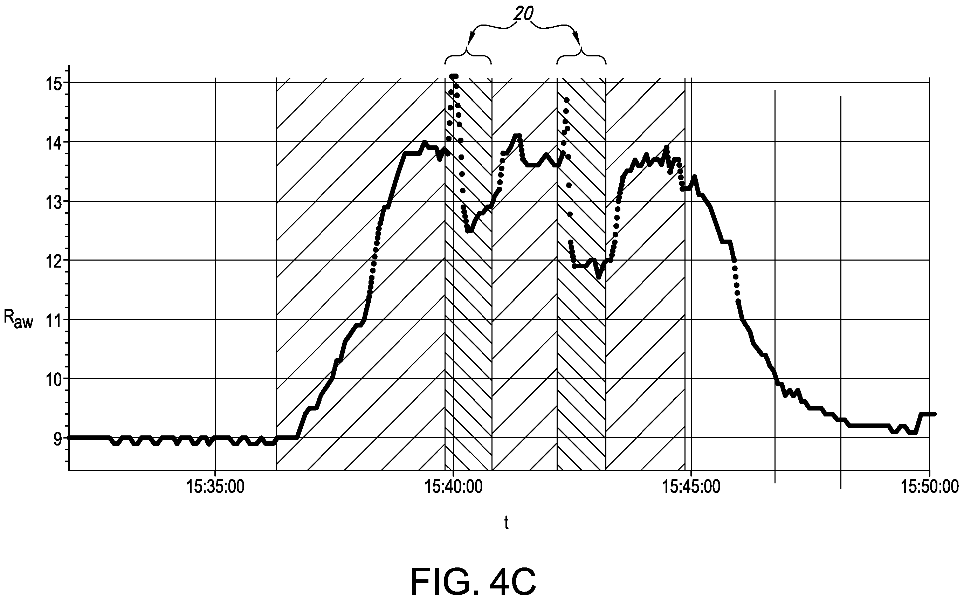

Fig. 4C schematically depicts bronchoconstriction in sheep by $CO_2$ exposure (6% fraction mixed with 94% oxygen) evidenced by an increase in the airway resistance $R_{aw}$ (after deduction of resistance from the ventilator system and tubing) during $CO_2$ exposure (hatched area - both /-hatched and \-hatched areas). Specifically, Fig. 4C schematically depicts the development of $R_{aw}$ over time t (in h:m:s). $R_{aw}$ is observed to increase by about 5 cm $H_2O$ /L/sec. HF blocks according to the method of the invention were performed twice by providing electrical signals 20 (\-hatched area) during this period of heightened resistance, which electrical signals reduced induced $R_{aw}$ by about 20-40%. Both provided electrical signals had the following parameters: CPT - 1.465 mC/s; Amplitude $A_E$ - 6 mA; duration $T_D$ of 20 $\mu$s; frequency of 12207 Hz; signal duration $T_E$ - 60 s. The two electrical signals 20 were provided with a first electrode 221 and a second electrode 222 of RevD3 (see above) for the first electrical stimulation and with a first electrode 221 and a second electrode 222 of RevD2 for the second electrical stimulation, wherein RevD2 and RevD3 were arranged at different locations along the nerve. This may be considered a high effectivity of the block, compared for example with tiotropium, a common long-acting muscarinic antagonist, for which >100-fold doses of the human marketed dose are needed to achieve similar effects in this model.

Hence, in embodiments, the electrical signal may be configured to (or "parameterized to") block nerve conduction in a pulmonary B-fiber such that airway resistance in the subject 50 is decreased.

Fig. 4D shows a profiler view corresponding to a statistical model (n=99 block attempts during $CO_2$ induced bronchoconstriction from a total of n=20 experiments; instances excluded with block effects >105% and lower than -5%). Specifically, Fig. 4D depicts how the block effect $B_E$ (in % signal block) and two types of onset effect $O_E$ (in % signal increase) would vary with changing values of the x factors: CPT (spline modelled) and frequency $F_E$ (log transformed). The block effect $B_E$ indicates the resistance block activity, onset effect $O_{E\_R}$ indicates a respiratory onset effect, and onset effect $O_{E\_C}$ indicates a cardiac onset effect. The highest block appears to be obtained at around 1.5 CPT. Of note: In the statistical regression model shown in Fig. 4D (and in Fig. 4B; see above) CPT was modeled as a spline rather than as a line. This spline was created by (automatically) placing suitable anchor points (or knots). For the model depicted in Fig. 4D (related to $CO_2$ exposure), the knot point with the highest CPT value (placed at 1.58192) was not significantly lower than the mean (p=0.15) (see also width of the confidence interval for higher CPT values), meaning that an upper limit of efficacy of CPT values cannot be established in the $CO_2$ blocking model and may lie well above 3 mC/s (and may thus lie at yet higher values for applications involving human B fibers, such as in the context of COPD; see discussion below).

The corresponding effect tests for the block and onset effects of Fig. 4D are shown in tables 6-8:

Effect test for block effect **$B_E$**

| Source | Nparm | DF | Sum of Squares | F Ratio | p-value |
|---|---|---|---|---|---|
| CPT&Knotted | 3 | 3 | 1982.7288 | 2.7330 | 0.0481* |
| Log($F_E$) | 1 | 1 | 23.8960 | 0.0988 | 0.7540 |

**Effect test for onset effect $O_{E\_R}$**

| Source | Nparm | DF | Sum of Squares | F Ratio | p-value |
|---|---|---|---|---|---|
| CPT&Knotted | 3 | 3 | 131.36260 | 0.7074 | 0.5500 |
| Log($F_E$) | 1 | 1 | 295.69498 | 4.7768 | 0.0313* |

**Effect test for onset effect $O_{E\_C}$**

| Source | Nparm | DF | Sum of Squares | F Ratio | p-value |
|---|---|---|---|---|---|
| CPT&Knotted | 3 | 3 | 584.60094 | 1.2236 | 0.3055 |
| Log($F_E$) | 1 | 1 | 725.81596 | 4.5574 | 0.0354* |

As in the cardiac block experiment (see above), higher frequencies $F_E$ were observed to significantly reduce onset effects in the $CO_2$-induced bronchoconstriction model (both cardiac and respiratory) ($p<0.05$). For CPT a similar initial slope to an efficient block as in the cardiac experiment was observed, but with a later transition point (~1.3 CPT) ($p<0.05$).

Without being bound by theory, it is expected that the observed difference in CPT needed for block effects in pulmonary versus cardiac B fibers is not due to a physiological difference in the respective B fibers but rather due to the extent of stimulation provided by the electrical stimulation of experiment 1, which selectively activates fibers that lie close to the stimulating electrode, in contrast to the $CO_2$ exposure of experiment 2, which presumably activates all B fibers in both vagus nerves. The expectation is that a higher CPT is therefore needed for the latter scenario.

Hence, the selected parameters for the high-frequency block were found suitable for blocking both cardiac and pulmonary efferent B-fibers in sheep. It will be known to the person skilled in the art that nerves from different species may show differences in anatomy/histology, which may in turn affect the parameters suitable eliciting or blocking action potential conduction in axons. This may be especially pronounced when stimulating from the surface of a nerve, such as with a cuff electrodes. Without being bound by theory, the main anatomical parameters pertinent to these issues may be the perineurium thickness and the packing density of nerve fascicles within a nerve, such as described in PELOT, NA et al., *Quantified morphology of the cervical and subdiaphragmatic vagus nerves of human, pig, and rat*, Frontiers in neuroscience, 2020, 14, 601479. For instance, stimulation thresholds of B-fibers from rat, pigs and humans as predicted by simulation model differ largely. MUSSELMAN, ED et al., *Validated computational models predict vagus nerve stimulation thresholds in preclinical animals and humans*, Journal of Neural Engineering, 2023, Vol. 20, number 3, describe that B-fiber thresholds of human nerve physiology is predicted around approximately 2 times the thresholds for pig, which is another well accepted large animal model for vagus nerve stimulation. Further, the thresholds for sheep nerves may generally be lower than those for pig nerves. As a consequence, CPT values suitable for human application may differ from those found suitable for application in sheep. In particular, without being bound by theory, the values for human application may be expected to be (about) a factor of two higher than those suitable for application in sheep.

In embodiments, the electrical signal may, especially during the therapeutic period, have a CPT $\geq 0.25$ mC/s, such as $\geq 0.75$ mC/s, especially $\geq 1.0$ mC/s. In further embodiments, the electrical signal may, especially during the therapeutic period, have a CPT $\geq 1.2$ mC/s, especially $\geq 1.4$ mC/s, such as $\geq 1.5$ mC/s, especially $\geq 1.6$ mC/s, such as $\geq 2.0$ mC/s. Especially, in such embodiments, the electrical signal may, especially during the therapeutic period, have a CPT $\leq 7.0$ mC/s, such as $\leq 6.5$ mC/s, especially $\leq 6.0$ mC/s, such as $\leq 5.5$ mC/s, especially $\leq 5.0$ mC/s. In further embodiments, the electrical signal may, especially during the therapeutic period, have a CPT $\leq 4.0$ mC/s, especially $\leq 3.0$ mC/s, such as $\leq 2.5$ mC/s, especially $\leq 2.0$ mC/s.

Fig. 5 schematically depicts an embodiment of an electrical signal 20 in amplitude A versus time t. In the depicted embodiment, the electrical signal 20 has frequency $F_E$ (inverse of a pulse period), amplitude $A_E$ and pulse widths $T_P$. the electrical 20 comprises a plurality of phases 30, wherein the plurality of phases 30 comprises alternating cathodic phases 31 and anodic phases 32.

In the depicted embodiment, the successive cathodic phases 31 and anodic phases 32 are charge-balanced, wherein each anodic phase 32 comprises providing a pulse 21, and wherein each cathodic phase 32 comprises providing a counter pulse 22 (may also be *vice versa*), wherein the amplitude peak or amplitude AUC or charge of the pulse and the counter pulse differ by less than 1%, and wherein the durations of the pulse and the counter pulse differ by less than 1%. Further, in the depicted embodiment, the electrical signal 20, especially the pulse and the counter pulse, have (symmetric) rectangular pulse shapes.

In the depicted embodiment, the electrical signal further comprises gap phases 33, wherein the gap phases 33 are interspersed between successive cathodic phases 31 and anodic phases 32, including between successive anodic phases 32 and thereupon following cathodic phases 31. The gap phases have gap durations $T_G$ individually selected from the range of 5 - 80 $\mu$s, especially from the range of 19 - 21 $\mu$s.

The term "plurality" refers to two or more. Furthermore, the terms "a plurality of" and "a number of" may be used interchangeably.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%. Moreover, the terms "about" and "approximately" may also relate to 90% or higher, such as 95% or higher, especially 99% or higher, even more especially 99.5% or higher, including 100%. For numerical values it is to be understood that the terms "substantially", "essentially", "about", and "approximately" may also relate to the range of 90% - 110%, such as 95%-105%, especially 99%-101% of the values(s) it refers to.

The term "comprise" also includes embodiments wherein the term "comprises" means "consists of".

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

The term "further embodiment" and similar terms may refer to an embodiment comprising the features of the previously discussed embodiment, but may also refer to an alternative embodiment.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", "include", "including", "contain", "containing" and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage.

The invention also provides a control system that may control the device, apparatus, or system, or that may execute the herein described method or process. Yet further, the invention also provides a computer program product, when running on a computer which is functionally coupled to or comprised by the device, apparatus, or system, controls one or more controllable elements of such device, apparatus, or system.

The invention further applies to a device, apparatus, or system comprising one or more of the characterizing features described in the description and/or shown in the attached drawings. The invention further pertains to a method or process comprising one or more of the characterizing features described in the description and/or shown in the attached drawings. Moreover, if a method or an embodiment of the method is described being executed in a device, apparatus, or system, it will be understood that the device, apparatus, or system is suitable for or configured for (executing) the method or the embodiment of the method, respectively.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

**Claims**

1.  A system (100) for inhibiting conduction of an action potential (25) in a nerve (10), wherein the system (100) comprises an arrangement (200), wherein:

    - the arrangement (200) comprises a tubular hosting element (211) configured for at least partially surrounding the nerve (10), wherein the tubular hosting element (211) comprises a plurality of electrodes (220);
    - the system (100) is configured to apply in a treatment mode of the system (100) an electrical signal (20) via at least part of the plurality of electrodes (220), wherein the following applies:

        - the electrical signal (20) has a frequency ($F_E$) selected from the range of 5 - 50 kHz;
        - the electrical signal (20) has an amplitude ($A_E$) selected from the range of 2 - 10 mA;
        - the electrical signal (20) comprises a plurality of phases (30), wherein the plurality of phases (30) comprises alternating cathodic phases (31) and anodic phases (32), wherein each phase (30) has a duration $T_P$ individually selected from the range of 5 - 60 $\mu$s; and
        - the electrical signal (20) comprises a therapeutic period, wherein $F_E$, $A_E$ and $T_P$ are constant during the therapeutic period, and wherein during the therapeutic period the electrical signal (20) has a charge per time (CPT) selected from the range of 1.0 - 7.0 mC/s, wherein CPT = $F_E*A_E*T_P$.

2.  The system (100) according to claim 1, wherein the electrical signal (20) has a charge per time (CPT) selected from the range of 1.0 - 3.0 mC/s.

3.  The system (100) according to any one of the preceding claims, wherein the system (100) is for preventing and/or treating disease in a subject (50), wherein the disease comprises a respiratory disease selected from the group comprising chronic obstructive pulmonary disease, asthma, sleep apnea, asthma-COPD overlap syndrome, cystic fibrosis, interstitial lung disease, lung fibrosis, and pulmonary hypertension, and wherein the action potential is related to one or more of bronchoconstriction, mucus hypersecretion and inflammation.

4.  The system (100) according to claim 3, wherein the disease comprises comprising chronic obstructive pulmonary disease.

5.  The system (100) according to any one of the preceding claims 1-2, wherein the system (100) is for preventing and/or treating a disease in a subject (50), wherein the disease comprises a cardiac disease selected from the group comprising atrial fibrillation, symptomatic clinical bradyarrhythmia, vasovagal syncope, sinus node dysfunction, and functional atrioventricular block.

6.  The system (100) according to any one of the preceding claims, wherein the tubular hosting element (211) has a diameter $D_H$, and wherein along a longitudinal axis of the tubular hosting element each electrode (220) has a length (L) individually selected from the range of $0.1*D_H$ - $0.5*D_H$, and wherein circumferentially around the longitudinal axis (A) each electrode (220) has a width (W) individually selected from the range of $0.1*D_H$ - $0.75*D_H$.

7.  The system (100) according to claim 6, wherein the electrodes (220) comprise first electrodes (221) and second electrodes (222), wherein the first electrodes (221) are circumferentially arranged in the tubular hosting element in a first electrode array (231), and wherein the second electrodes (222) are circumferentially arranged in the tubular hosting element in a second electrode array (232), wherein the first electrode array (231) and the second electrode array (232) are separated by a first edge-to-edge distance ($d_1$) along the longitudinal axis (A), wherein the first edge-to-edge distance ($d_1$) is selected from the range of 0.5 - 2 mm.

8.  The system (100) according to claim 7, wherein the electrodes (220) further comprise third electrodes (223), wherein the third electrodes (223) are circumferentially arranged in the tubular hosting element in a third electrode array (233), wherein the second electrode array (232) is arranged between the first electrode array (231) and the third electrode array (233), and wherein the second electrode array (232) and the third electrode array (233) are separated by a second edge-to-edge distance ($d_2$) along the longitudinal axis (A), wherein the second edge-to-edge distance ($d_2$) is selected from the range of 0.5 mm - 3 mm.

9.  The system (100) according to any one of the preceding claims 7-8, wherein for each first electrode (221) applies that: the first electrode is longitudinally aligned with a second electrode (222), wherein surface areas of the first electrode (221) and of the second electrode (222) differ by less than 5%.

10. The system (100) according to any one of the preceding claims 7-9, wherein the first electrode array (231) comprises $n_1$ first electrodes (221), and wherein the second electrode array (232) comprises $n_2$ second electrodes, wherein $n_1$ is selected from the range of 4-12, and wherein $n_1 = n_2$.

11. The system (100) according to any one of the preceding claims, wherein the nerve (10) comprises a vagus nerve (11), and wherein the treatment mode comprises applying the electrical signal (20) to at least one B-fiber in the nerve (10).

12. The system (100) according to any one of the preceding claims, wherein the electrical signal (20) has a frequency $F_E$ selected from the range of 10 - 35 kHz, wherein the electrical signal (20) has an amplitude $A_E$ selected from the range of 5 - 8 mA, and wherein for each phase (30) the duration $T_P$ is individually selected from the range of 15 - 50 $\mu$s.

13. The system (100) according to any of the preceding claims, wherein the electrical signal (20) has a signal duration $T_E$, wherein $T_E$ is selected from the range of 10 s - 12 h.

14. The system (100) according to any one of the preceding claims, wherein the electrical signal (20) has a square pulse shape.

15. A method for inhibiting conduction of an action potential (25) in a nerve (10), wherein the method comprises providing an electrical signal (20) to the nerve (10) via one or more electrodes (220) wherein:

   - the electrical signal (20) has a frequency ($F_E$) selected from the range of 5 - 50 kHz;
   - the electrical signal (20) has an amplitude ($A_E$) selected from the range of 2 - 10 mA;
   - the electrical signal (20) comprises a plurality of phases (30), wherein the plurality of phases (30) comprises alternating cathodic phases (31) and anodic phases (32), wherein each phase has a duration $T_P$ individually selected from the range of 5 - 60 $\mu$s;
   - the electrical signal has a charge per time (CPT) selected from the range of 1.0 - 7.0 mC/s, wherein CPT = $F_E * A_E * T_P$.

16. The method according to claim 15, wherein the electrical signal (20) has a charge per time (CPT) selected from the range of 1.0 - 3.0 mC/s.

17. The method according to any one of the preceding claims 15-16, wherein the method is for preventing and/or treating disease in a subject (50), wherein the disease comprises a respiratory disease selected from the group comprising chronic obstructive pulmonary disease, asthma, sleep apnea, asthma-COPD overlap syndrome, cystic fibrosis, interstitial lung disease, lung fibrosis, and pulmonary hypertension, and wherein the action potential is related to one or more of bronchoconstriction, mucus hypersecretion and inflammation.

18. The method according to any one of the preceding claims 15-16, wherein the method is for preventing and/or treating a disease in a subject (50), wherein the disease comprises a cardiac disease selected from the group comprising atrial fibrillation, symptomatic clinical bradyarrhythmia, vasovagal syncope, sinus node dysfunction, and functional atrioventricular block.

19. The method according to any one of the preceding claims 15-18, wherein the nerve (10) comprises a vagus nerve (11), and wherein the method comprises applying the electrical signal (20) to the nerve (10) to stimulate a B-fiber in the nerve (10).

20. The method according to any one of the preceding claims 15-19, wherein the electrical signal (20) has a frequency ($F_E$) selected from the range of 10 - 35 kHz, and wherein the electrical signal (20) has an amplitude ($A_E$) selected from the range of 5 - 8 mA, and wherein for each phase (30) the duration $T_P$ is individually selected from the range of 15 - 50 $\mu$s.

21. The method (100) according to any of the preceding claims 15-20, wherein the electrical signal (20) has a signal duration $T_E$, wherein $T_E$ is selected from the range of 10 s - 12 hours.

22. The method according to any one of the preceding claims 15-21, wherein the electrical signal (20) has a square pulse shape.

FIG. 1A

FIG. 1B

FIG. 1C

FIG. 2

FIG. 3

FIG. 4A

EP 4 635 557 A1

FIG. 4B

FIG. 4C

EP 4 635 557 A1

EP 4 635 557 A1

FIG. 4D

FIG. 5

EP 4 635 557 A1

Europäisches
Patentamt

European
Patent Office

Office européen
des brevets

# EUROPEAN SEARCH REPORT

**Application Number**

EP 24 17 0565

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/224989 A1 (SCHEPIS ERIC A [US] ET AL) 10 August 2017 (2017-08-10) * paragraphs [0036], [0110] - [0113]; figures 1-4 * | 1-22 | INV. A61N1/36 ADD. A61N1/05 |
| A | US 2019/321640 A1 (CARMENA JOSE M [US] ET AL) 24 October 2019 (2019-10-24) * paragraphs [0062], [0092], [0131], [0194] - paragraphs [0413], [0414] * | 1-22 | |

**TECHNICAL FIELDS SEARCHED (IPC)**

A61N
A61B

The present search report has been drawn up for all claims

| Place of search | Date of completion of the search | Examiner |
|---|---|---|
| Munich | 7 October 2024 | Edward, Vinod |

EPO FORM 1503 03.82 (P04C01)

EP 4 635 557 A1

## ANNEX TO THE EUROPEAN SEARCH REPORT
## ON EUROPEAN PATENT APPLICATION NO.

EP 24 17 0565

This annex lists the patent family members relating to the patent documents cited in the above-mentioned European search report.
The members are as contained in the European Patent Office EDP file on
The European Patent Office is in no way liable for these particulars which are merely given for the purpose of information.

07-10-2024

| Patent document cited in search report | Publication date | Patent family member(s) | Publication date |
|---|---|---|---|
| US 2017224989 A1 | 10-08-2017 | AU 2015306890 A1 | 09-03-2017 |
| | | BR 112017004047 A2 | 05-12-2017 |
| | | CA 2959330 A1 | 03-03-2016 |
| | | CN 106714898 A | 24-05-2017 |
| | | CN 117482396 A | 02-02-2024 |
| | | EP 3185951 A2 | 05-07-2017 |
| | | JP 6902464 B2 | 14-07-2021 |
| | | JP 2017525495 A | 07-09-2017 |
| | | KR 20170047300 A | 04-05-2017 |
| | | MX 370944 B | 10-01-2020 |
| | | RU 2017105405 A | 27-09-2018 |
| | | US 2017224989 A1 | 10-08-2017 |
| | | WO 2016032929 A2 | 03-03-2016 |
| US 2019321640 A1 | 24-10-2019 | AU 2019255373 A1 | 26-11-2020 |
| | | BR 112020021286 A2 | 26-01-2021 |
| | | CA 3097139 A1 | 24-10-2019 |
| | | CN 112351812 A | 09-02-2021 |
| | | CN 118454103 A | 09-08-2024 |
| | | EP 3781255 A1 | 24-02-2021 |
| | | IL 278031 A | 30-11-2020 |
| | | IL 312444 A | 01-06-2024 |
| | | JP 2021522032 A | 30-08-2021 |
| | | JP 2024056709 A | 23-04-2024 |
| | | KR 20210016346 A | 15-02-2021 |
| | | US 2019321640 A1 | 24-10-2019 |
| | | US 2024017071 A1 | 18-01-2024 |
| | | WO 2019204769 A1 | 24-10-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10441787 B2

**Non-patent literature cited in the description**

- **VARGA I ; MRAVEC B**. Nerve Fiber Types, Nerves and Nerve Injuries. Academic Press, 2015, 107-113
- **PELOT, NA et al.** Quantified morphology of the cervical and subdiaphragmatic vagus nerves of human, pig, and rat. *Frontiers in neuroscience*, 2020, vol. 14, 601479
- **MUSSELMAN, ED et al.** Validated computational models predict vagus nerve stimulation thresholds in preclinical animals and humans. *Journal of Neural Engineering*, 2023, vol. 20 (3)